# EUROPEAN PATENT APPLICATION

(11) **EP 4 779 312 A1**
(43) Date of publication of application: **22.07.2026**
(21) Application number: 24865471.7
(22) Date of filing: 11.09.2024
(51) Int. Cl.: G01N 33/68

(54) **METHOD FOR ASSISTING DIAGNOSIS OF GASTROINTESTINAL DISEASE, METHOD FOR SCREENING DIAGNOSTIC MARKER FOR GASTROINTESTINAL DISEASE, AND REAGENT AND KIT FOR DETECTING GASTROINTESTINAL DISEASE-RELATED PROTEIN**

(30) Priority: 12.09.2023 JP 2023147763
(71) Applicant: Eiken Kagaku Kabushiki Kaisha, Tokyo 101-0062 (JP)
(72) Inventor: MURAKAMI Yusuke, Shimotsuga-gun, Tochigi 329-0114 (JP); SAKAMAKI Nozomi, Shimotsuga-gun, Tochigi 329-0114 (JP)
(74) Representative: J A Kemp LLP
(86) International application number: PCT/JP2024/032513
(87) International publication number: WO 2025/057971

(57) **Abstract**

A method for assisting diagnosis of a gastrointestinal disease, comprising:
a step of separating fecal exosomes from feces of a subject, and
a step of detecting a gastrointestinal disease-related protein from the separated fecal exosomes,
wherein the gastrointestinal disease-related protein is a gastrointestinal disease-related protein that satisfies any one of the following conditions (a) and (b):
(a) a content ratio relative to a total amount of the fecal exosomes is 1.5 times or more a content ratio relative to a total amount of the feces,
(b) the protein is detected only from the separated fecal exosomes and is substantially not detected from the feces.

## Description

### [Technical Field]

The present invention relates to a method for assisting diagnosis of a gastrointestinal disease, a method for screening a diagnostic marker for a gastrointestinal disease, and a detection reagent and kit for a gastrointestinal disease-related protein, and more specifically, to a method for assisting diagnosis of a gastrointestinal disease, and a method for screening a diagnostic marker for a gastrointestinal disease, as well as a detection reagent for a gastrointestinal disease-related protein for use in these methods and a kit containing the same.

### [Background Art]

In recent years, as methods for assisting the diagnosis of various diseases, testing methods that detect and quantify target substances such as circulating tumor cells, cell-free DNA, exosomes, and proteins derived therefrom as biomarkers for various diseases from liquid specimens such as blood specimens and urine specimens have been widely adopted. These testing methods using liquid specimens are useful as minimally invasive testing methods that are less burdensome on the body compared to tissue examinations and the like, and because they can be performed multiple times, in recent years, they have been mainly expected to be utilized not only for the diagnosis of tumor-related diseases but also for the selection of treatment methods, follow-up observations thereof, and the like. However, in the liquid specimens described above, there was a problem that the amount of biomarker per total specimen amount is generally very small, and biomarkers derived from various tissues and organs are mixed, making it difficult to detect the biomarker of the target disease with high sensitivity. For example, although it is known that extracellular vesicles such as exosomes contain marker proteins effective for distinguishing diseases such as cancer, Takuya Naito et al., FEBS Letters, Vol. 595, 19, 2021, p. 2522-2532 (Non-Patent Literature 1) reports that it is difficult to identify which organ or tissue is the source of origin of marker proteins contained in extracellular vesicles of a blood specimen.

As a minimally invasive testing method, in addition to the liquid specimens described above, there is also a method that assists in the diagnosis of various diseases by using feces as a specimen and detecting and quantifying marker proteins for the diseases therefrom. For example, International Publication No. WO 2013/162368 (Patent Literature 1) describes a method for screening for colorectal cancer by detecting a plurality of marker proteins from a sample of homogenized feces.

### [Citation List]

### [Patent Literature]

[PTL 1] International Publication No. WO 2013/162368 [Non Patent Literature]
[NPL 1] Takuya Naito et al., FEBS Letters, Vol. 595, 19 , 2021, p. 2522-2532

### [Summary of Invention]

### [Technical Problem]

As described above, feces are useful as a minimally invasive specimen. However, because feces contain more contaminants than liquid specimens, there was a problem that the relative content of the target marker protein becomes small and falls below the detection limit. Furthermore, when detecting a marker protein related to a gastrointestinal disease, feces contain many proteins derived from other tissues, particularly the liver and liver diseases, etc., in addition to the target marker protein (Patent Literature 1); therefore, in order to detect such a target marker protein from feces, it was necessary to employ a specific purification method and a high-sensitivity detection method for each of the marker proteins.

The present invention has been made in view of the problems of the conventional art described above, and an object of the present invention is to provide a method for assisting diagnosis of a gastrointestinal disease and a method for screening a diagnostic marker for a gastrointestinal disease that can efficiently detect a gastrointestinal disease-related protein using feces as a specimen, as well as a detection reagent and kit for a gastrointestinal disease-related protein for use in these methods.

### [Solution to Problem]

The present inventors conducted intensive research to achieve the above object, fractionated exosomes from feces, and conducted various analyses/examinations on the proteins contained in the fractionated fecal exosomes. As a result, they discovered a novel phenomenon that within fecal exosomes, gastrointestinal disease-related proteins, including proteins derived from the gastrointestinal tract, exist at a high frequency, specifically compared to other proteins, at a higher content ratio than the content ratio relative to the total feces (= are concentrated) (in this specification, this phenomenon is sometimes referred to as "concentration"), and also confirmed that such "concentration" of gastrointestinal disease-related proteins in fecal exosomes occurs statistically significantly. The phenomenon that the gastrointestinal disease-related proteins are concentrated at a high frequency in fecal exosomes is a surprising finding, in view of the fact that it is difficult to identify the source of origin of proteins contained in exosomes and the like in the blood specimens described above. Therefore, they found that by separating such fecal exosomes from feces and detecting the gastrointestinal disease-related proteins "concentrated" within such fecal exosomes, it becomes possible to detect the target gastrointestinal disease-related protein extremely efficiently without using a specific purification method or a high-sensitivity detection method. Furthermore, because the gastrointestinal disease-related proteins concentrated therein are detected after separating the exosomes, it becomes possible to detect the target gastrointestinal disease-related protein even by Western blotting or the like, and it also becomes possible to efficiently perform the diagnosis of a related gastrointestinal disease using the presence/absence of detection or the amount of the target gastrointestinal disease-related protein as an indicator, which led to the completion of the present invention.

The aspects of the present invention, based on such findings, are as follows.
[1] A method for assisting diagnosis of a gastrointestinal disease, comprising:
   a step of separating fecal exosomes from feces of a subject, and
   a step of detecting a gastrointestinal disease-related protein from the separated fecal exosomes,
   wherein the gastrointestinal disease-related protein is a gastrointestinal disease-related protein that satisfies any one of the following conditions (a) and (b):
      (a) a content ratio relative to a total amount of the fecal exosomes is 1.5 times or more a content ratio relative to a total amount of the feces,
      (b) the protein is detected only from the separated fecal exosomes and is substantially not detected from the feces.
[2] The method according to [1], wherein the gastrointestinal disease-related protein is at least one selected from the group consisting of OLFM4, LGALS3BP, LAMP1, ATP5F1A, DLD, ENPP4, MAPK1, PCYOX1, SMPD3, VILL, and RPS7.
[3] The method according to [1] or [2], wherein the gastrointestinal disease is an intestinal disease.
[4] The method according to any one of [1] to [3], wherein the gastrointestinal disease is colorectal cancer.
[5] The method according to any one of [1] to [4], further comprising a step of quantifying the detected gastrointestinal disease-related protein.
[6] A method for assisting diagnosis of a gastrointestinal disease, comprising:
   a step of separating fecal exosomes from feces of a subject, and
   a step of detecting a gastrointestinal disease-related protein from the separated fecal exosomes,
   wherein the gastrointestinal disease-related protein is at least one selected from the group consisting of ZMPSTE24, YWHAG, UGDH, TPM4, TC2N, SUSD2, STOM, ST6GALNAC1, SPTBN1, SLC6A14, SLC46A1, SLC15A4, SERPINF2, SDHB, SDHA, SAFB2, RPS16, RNF128, RNASE3, RAB21, RAB14, PRKG2, PLEC, PLA2G10, NPEPPS, MYH14, MT-CYB, MAOA, ITGA3, IGLV7-46, HSD17B4, HARS1, H1-4, GSTA1, GMDS, GFM2, FUT3, FLOT2, DNPEP, CNDP2, CLTC, B3GNT7, B2M, ATP9A, ARPC2, AP2A1, AP1B1, AGR2, ACTR2, ABRACL, YWHAH, WDR1, VILL, VAMP8, TUBB4B, TSPAN6, TPP1, TPM3, TPM1, TMEM30A, SYPL1, SQOR, SPPL2A, SMPD3, SLC9A3, SLC44A1, SLC2A5, SLC25A5, SLC15A1, SERPINA7, SCARB2, S100A11, RPS7, RPS3, RPS23, RPS10, RPL30, RNPEP, RHPN2, RAB3D, RAB32, RAB2A, PTPN6, PSMB8, PSMB1, PSMA1, PRKCD, PRKACA, PRDX6, PRDX5, PRDX1, PPP1CB, PLA2G2A, PKM, PICALM, PGK1, PGD, PFN1, PCYOX1, PCBP1, P2RX4, NIBAN2, NEDD4L, NCSTN, MYOF, MYO1E, MYO1C, MYO1B, MYH9, MUC4, MUC3A, MGST3, MGLL, MEP1A, LYN, LGALS4, LGALS3BP, LDHA, LAMP1, IGKV1-39, IGHM, IGHG2, IDH1, HSPA5, HSP90AB1, HNRNPA2B1, HLA-DMA, H2BC12, GSTP1, GRN, GPI, GPD2, GPA33, GP2, GNAI2, GHITM, GDPD3, GAPDH, FLOT1, F11R, EPCAM, ENPP4, ENOl, EIF5A, EIF4A1, EEF2, EBP, DSTN, DLD, CYFIP1, CSNK1A1, CPNE3, CLIC1, CHMP2A, CEACAM5, CEACAM1, CD151, CAPZB, CAPN1, CAP1, C9, ATP1B1, ATP1A1, ASAH1, ARL1, ARHGDIA, ANXA4, ANXA3, ALDOA, ALDH3B1, ACTN4, ACAA1, XPO1, VPS26A, UQCRC2, UQCRC1, UBA1, TPP2, TMEM33, TMEM205, TMED4, TMED2, TMED10, TM9SF3, TM9SF2, THEMIS2, TARDBP, SYNGR2, SYK, STIP1, STAT3, STAT1, SSR4, SRSF3, SPCS3, SPAST, SOD2, SLC3A2, SLC2A3, SLC2A1, SLC16A3, SIRPA, SIL1, SERPINB5, SELENOP, SEC31A, SEC23A, RPS4X, RPS3A, RPS18, RPN1, RPL7, RPL6, RPL5, RPL4, RPL21, RPL13A, RARS1, QARS1, PTK2B, PTGES3, PSMD3, PSMD12, PSMD1, PSMC2, PRXL2A, PRPS1, PRKDC, PFKP, PCK2, PC, PAICS, OCIAD2, OAF, NT5C2, NIPSNAP2, NDUFB10, MT-CO2, MOB1B, MICAL1, MGST1, MCCC2, M6PR, LNPEP, LBR, KDELR2, KDELR1, KARS1, ITGAV, IMPDH2, IGKV1-12, IDH3B, IDH2, HTT, HSPA4, HSD17B10, HNRNPUL2, HNRNPU, HNRNPK, HMGCS2, HLA-B, HK1, HIBCH, HADHA, H1-0, GRTP1, GPD1, GMPPB, GCLM, GBE1, GARS1, GALNT2, FGR, FCN3, FASN, ERO1A, ENTPD1, EML4, EIF3D, DYNC1H1, DNM2, DLAT, DHX9, DHRS7, DARS1, CYB5B, CSK, CRYL1, CPT1A, COX5B, CORO1C, COPG1, COPA, CLDN7, CHST5, CCT4, CCT3, C8G, BSG, B3GNT3, ATP6V0D1, ATP5MG, ARPC3, ARPC1B, ARHGAP1, APMAP, AKR1C3, ADSS2, ADGRG3, ADGRE5, ACSL1, ACADM, VPS35, TMED9, TMED7, TM9SF4, STXBP6, SLC25A11, SIGMAR1, SF3B3, RPS9, RPS24, RPS13, RPLPO, RPL3, RPL18, RACK1, PYGB, PPP2CB, PPIB, PLTP, PHB2, NOSTRIN, NARS1, MAN1A1, LTA4H, JAKMIP3, ITGA2, HSD17B11, HNRNPC, GOT2, GANAB, G6PD, ERAP1, EEF1G, DOP1B, DHRS9, COPB2, ATP5MF, ATP2A2, ATIC, ARCN1, APRT, APIP, AP1S1, ALDH9A1, ADAM10, ACSL5, ACLY, TKT, SERPINA6, RPL14, PARP4, MFGE8, MAPK1, HSP90B1, GPLD1, DDC, C8B, C7, ATP5F1A, ACAA2, LCP1, ANXA1, OLFM4, FBLN1, COL6A1, TGFBI, and THBS1.
[7] The method according to [6], wherein the gastrointestinal disease-related protein is at least one selected from the group consisting of OLFM4, LGALS3BP, LAMP1, ATP5F1A, DLD, ENPP4, MAPK1, PCYOX1, SMPD3, VILL, and RPS7.
[8] The method according to [6] or [7], wherein the gastrointestinal disease is an intestinal disease.
[9] The method according to any one of [6] to [8], wherein the gastrointestinal disease is colorectal cancer.
[10] The method according to any one of [6] to [9], further comprising a step of quantifying the detected gastrointestinal disease-related protein.
[11] A detection reagent for the gastrointestinal disease-related protein for use in the method according to any one of [1] to [10].
[12] A kit for use in the method according to any one of [1] to [10], comprising a detection reagent for the gastrointestinal disease-related protein.
[13] A method for screening a diagnostic marker for a gastrointestinal disease, comprising:
   a step of separating fecal exosomes from feces,
   a step of detecting a protein from the separated fecal exosomes, and
   a step of selecting a candidate for a diagnostic marker for a gastrointestinal disease from the detected protein, using as an indicator that the protein satisfies any one of the following conditions (a) and (b):
      (a) a content ratio relative to a total amount of the fecal exosomes is 1.5 times or more a content ratio relative to a total amount of the feces,
      (b) the protein is detected only from the separated fecal exosomes and is substantially not detected from the feces.
[14] The screening method according to [13],
   wherein the step of separating fecal exosomes from the feces and the step of detecting a protein from the separated fecal exosomes are each performed in a colorectal cancer patient and a healthy subject, and
   the step of selecting a candidate for a diagnostic marker for the gastrointestinal disease is a step of selecting a candidate for a diagnostic marker for a gastrointestinal disease from the detected protein, using as an indicator that the protein satisfies any one of the following conditions (c) and (d):
      (c) satisfies any one of the conditions (a) and (b) in a colorectal cancer patient,
      (d) satisfies any one of the conditions (a) and (b) in a healthy subject.
[15] An immunological detection method for a gastrointestinal disease-related protein, comprising:
   a step of separating fecal exosomes from feces, and
   a step of immunologically detecting a gastrointestinal disease-related protein from the separated fecal exosomes.

### [Advantageous Effects of Invention]

According to the present invention, a gastrointestinal disease-related protein can be efficiently detected using feces as a specimen. Furthermore, according to the present invention, it is possible to provide a method for assisting diagnosis of a gastrointestinal disease and a method for screening a diagnostic marker for a gastrointestinal disease that can efficiently detect a gastrointestinal disease-related protein using feces as a specimen, as well as a detection reagent and kit for a gastrointestinal disease-related protein for use in these methods.

### [Brief Description of Drawings]

[Fig. 1] is a graph showing the relationship between the luminescence intensity by BLEIA (CD9/CD63 luminescence intensity, left axis, line graph) and each fraction (Fraction_1 to 12), and the relationship between the total protein amount (Protein (ng/µL), right axis, bar graph) and each fraction (Fraction_1 to 12), obtained in "(2) Detection of fecal exosomes by BLEIA" of Test Example 1.
[Fig. 2] is a diagram showing the results of Western blotting of Fraction_1 to 12, obtained in "(3) Detection of fecal exosomes by Western blotting" of Test Example 1.
[Fig. 3] is a graph showing the particle size distribution in Fraction_5 to 9, obtained in "(4) Detection of fecal exosomes by NanoSight" of Test Example 1.
[Fig. 4] is a plot diagram showing the correlation between the quantified value of proteins in healthy human fecal exosomes and the quantified value of proteins in healthy human total feces, obtained in "(1) Proteome analysis by mass spectrometry" of Test Example 2.
[Fig. 5] is a graph showing the relationship between the luminescence intensity by BLEIA (CD9/CD63 luminescence intensity, left axis, line graph) and each fraction (Fraction_1 to 12), and the relationship between the total protein amount by BCA protein assay (Protein (ng/µL), right axis, bar graph) and each fraction (Fraction_3 to 8), obtained in "(1) Purification of fecal exosomes from feces of colorectal cancer patient" of Test Example 3.
[Fig. 6] is a diagram showing the results of Western blotting of Fraction_3 to 8, obtained in "(1) Purification of fecal exosomes from feces of colorectal cancer patient" of Test Example 3.
[Fig. 7] is a graph showing the particle size distribution in Fraction_3 to 8, obtained in "(1) Purification of fecal exosomes from feces of colorectal cancer patient" of Test Example 3.
[Fig. 8] is a plot diagram showing the correlation between the quantified value of proteins in colorectal cancer patient fecal exosomes and the quantified value of proteins in healthy human fecal exosomes, obtained in "(2) Proteome analysis by mass spectrometry" of Test Example 3.
[Fig. 9] is a diagram showing proteins among the gastrointestinal disease-related proteins described in Tables 10 to 12 that were commonly detected from fecal exosomes in two or more colorectal cancer patient specimens, obtained in Test Example 5.
[Fig. 10] is a diagram showing proteins among the gastrointestinal disease-related proteins described in Tables 13 to 15 that were commonly detected from fecal exosomes in two or more colorectal cancer patient specimens, obtained in Test Example 5.
[Fig. 11] is a diagram showing proteins among the gastrointestinal disease-related proteins described in Tables 10 to 15 that were commonly detected from fecal exosomes in all colorectal cancer patient specimens (4 specimens), obtained in Test Example 5.

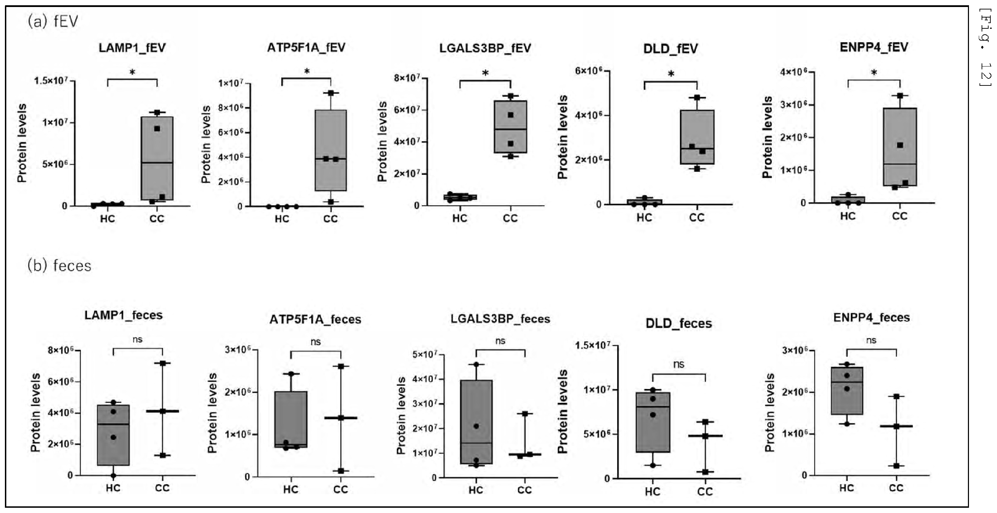

[Fig. 12] is a set of graphs for five types of colorectal cancer-related proteins (LAMP1, ATP5F1A, LGALS3BP, DLD, ENPP4) for which the quantified value in colorectal cancer patient fecal exosomes (fEV) was two times or more compared to the quantified value in colorectal cancer patient total feces (feces), obtained in Test Example 6, showing (a) a graph comparing the quantified value in healthy human fecal exosomes (HC) and the quantified value in colorectal cancer patient fecal exosomes (CC), respectively, and (b) a graph comparing the quantified value in healthy human total feces (HC) and the quantified value in colorectal cancer patient total feces (CC), respectively.

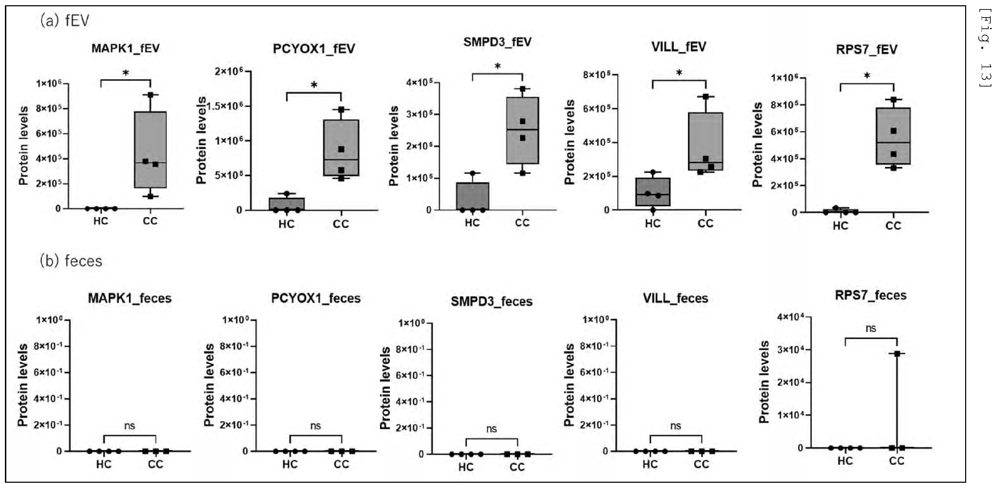

[Fig. 13] is a set of graphs for five types of colorectal cancer-related proteins (MAPK1, PCYOX1, SMPD3, VILL, RPS7) that were not detected in colorectal cancer patient total feces (feces) but were detected only in colorectal cancer patient fecal exosomes (fEV), obtained in Test Example 6, showing (a) a graph comparing the quantified value in healthy human fecal exosomes (HC) and the quantified value in colorectal cancer patient fecal exosomes (CC), respectively, and (b) a graph comparing the quantified value in healthy human total feces (HC) and the quantified value in colorectal cancer patient total feces (CC), respectively.

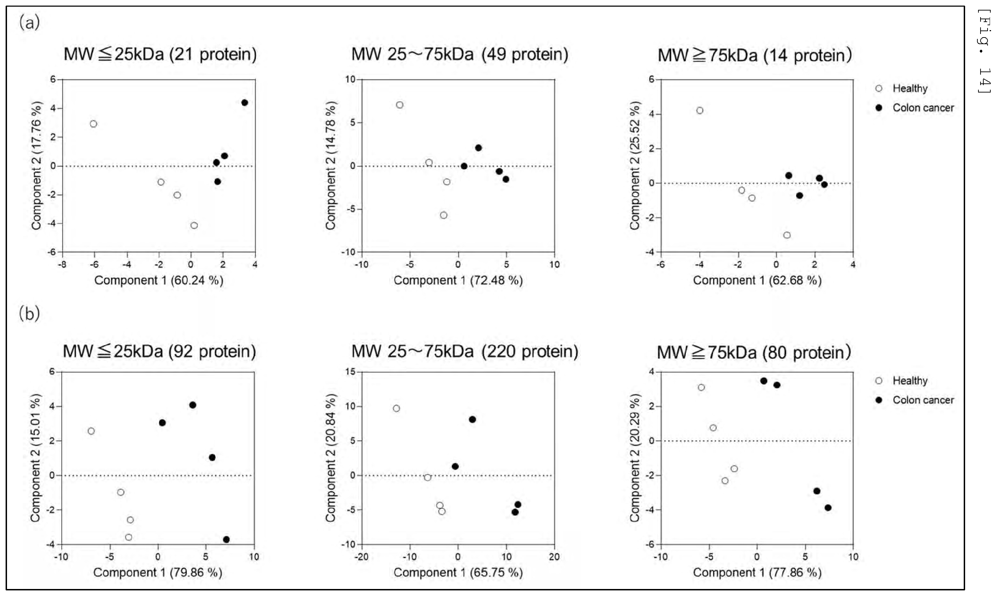

[Fig. 14] is a set of plot diagrams obtained in Test Example 7, showing the results of PLS regression analysis performed for each group: (a) for colorectal cancer-related proteins for which the quantified value in healthy human fecal exosomes was two times or more compared to the quantified value in healthy human total feces, using the quantified values in healthy human fecal exosomes (white circles, 4 specimens) and the quantified values in colorectal cancer patient fecal exosomes (black circles, 4 specimens), for the group with a molecular weight of 25 kDa or less (21 proteins), the group with a molecular weight of 25 to 75 kDa (49 proteins), and the group with a molecular weight of 75 kDa or more (14 proteins), and (b) for colorectal cancer-related proteins that were not detected in healthy human total feces but were detected only in healthy human fecal exosomes, using the quantified values in healthy human fecal exosomes (white circles, 4 specimens) and the quantified values in colorectal cancer patient fecal exosomes (black circles, 4 specimens), for the group with a molecular weight of 25 kDa or less (92 proteins), the group with a molecular weight of 25 to 75 kDa (220 proteins), and the group with a molecular weight of 75 kDa or more (80 proteins).

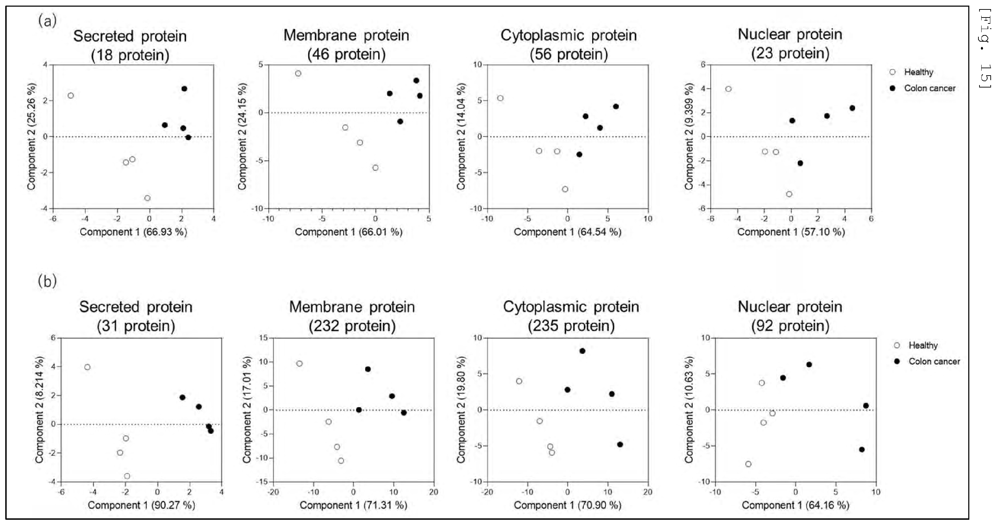

[Fig. 15] is a set of plot diagrams obtained in Test Example 7, showing the results of PLS regression analysis performed for each group: (a) for colorectal cancer-related proteins for which the quantified value in healthy human fecal exosomes was two times or more compared to the quantified value in healthy human total feces, using the quantified values in healthy human fecal exosomes (white circles, 4 specimens) and the quantified values in colorectal cancer patient fecal exosomes (black circles, 4 specimens), for the group of secreted proteins (Secreted protein, 18 proteins), the group of membrane proteins (Membrane protein, 46 proteins), the group of cytoplasmic proteins (Cytoplasmic protein, 56 proteins), and the group of nuclear proteins (Nuclear protein, 23 proteins), and (b) for colorectal cancer-related proteins that were not detected in healthy human total feces but were detected only in healthy human fecal exosomes, using the quantified values in healthy human fecal exosomes (white circles, 4 specimens) and the quantified values in colorectal cancer patient fecal exosomes (black circles, 4 specimens), for the group of secreted proteins (Secreted protein, 31 proteins), the group of membrane proteins (Membrane protein, 232 proteins), the group of cytoplasmic proteins (Cytoplasmic protein, 235 proteins), and the group of nuclear proteins (Nuclear protein, 92 proteins).

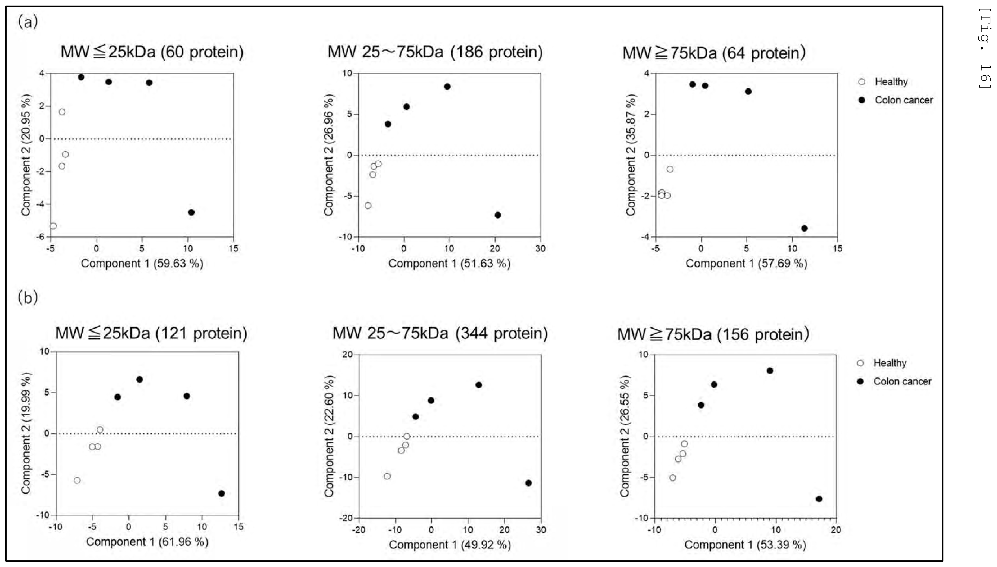

[Fig. 16] is a set of plot diagrams obtained in Test Example 7, showing the results of PLS regression analysis performed for each group: (a) for colorectal cancer-related proteins for which the quantified value in colorectal cancer patient fecal exosomes was two times or more compared to the quantified value in colorectal cancer patient total feces, using the quantified values in healthy human fecal exosomes (white circles, 4 specimens) and the quantified values in colorectal cancer patient fecal exosomes (black circles, 4 specimens), for the group with a molecular weight of 25 kDa or less (60 proteins), the group with a molecular weight of 25 to 75 kDa (186 proteins), and the group with a molecular weight of 75 kDa or more (64 proteins), and (b) for colorectal cancer-related proteins that were not detected in colorectal cancer patient total feces but were detected only in colorectal cancer patient fecal exosomes, using the quantified values in healthy human fecal exosomes (white circles, 4 specimens) and the quantified values in colorectal cancer patient fecal exosomes (black circles, 4 specimens), for the group with a molecular weight of 25 kDa or less (121 proteins), the group with a molecular weight of 25 to 75 kDa (344 proteins), and the group with a molecular weight of 75 kDa or more (156 proteins) .

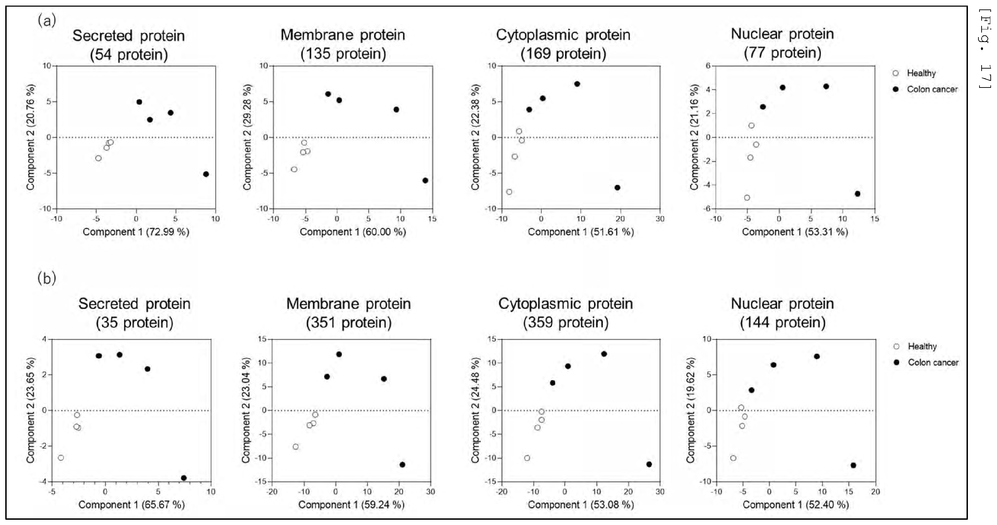

[Fig. 17] is a set of plot diagrams obtained in Test Example 7, showing the results of PLS regression analysis performed for each group: (a) for colorectal cancer-related proteins for which the quantified value in colorectal cancer patient fecal exosomes was two times or more compared to the quantified value in colorectal cancer patient total feces, using the quantified values in healthy human fecal exosomes (white circles, 4 specimens) and the quantified values in colorectal cancer patient fecal exosomes (black circles, 4 specimens), for the group of secreted proteins (Secreted protein, 54 proteins), the group of membrane proteins (Membrane protein, 135 proteins), the group of cytoplasmic proteins (Cytoplasmic protein, 169 proteins), and the group of nuclear proteins (Nuclear protein, 77 proteins), and (b) for colorectal cancer-related proteins that were not detected in colorectal cancer patient total feces but were detected only in colorectal cancer patient fecal exosomes, using the quantified values in healthy human fecal exosomes (white circles, 4 specimens) and the quantified values in colorectal cancer patient fecal exosomes (black circles, 4 specimens), for the group of secreted proteins (Secreted protein, 35 proteins), the group of membrane proteins (Membrane protein, 351 proteins), the group of cytoplasmic proteins (Cytoplasmic protein, 359 proteins), and the group of nuclear proteins (Nuclear protein, 144 proteins).
[Fig. 18] is a set of graphs for OLFM4 obtained in Test Example 9, showing (a) a graph comparing the quantified value in healthy human fecal exosomes (HC) and the quantified value in colorectal cancer patient fecal exosomes (CC), and (b) a graph comparing the quantified value in healthy human total feces (HC) and the quantified value in colorectal cancer patient total feces (CC).
[Fig. 19] is a diagram showing the results of detecting OLFM4, LAMP1, and LGALS3BP by Western blotting for healthy human fecal exosomes (HC) and colorectal cancer patient fecal exosomes (CC), obtained in Test Example 10.

### [Description of Embodiments]

Hereinafter, the present invention will be described in detail with reference to preferred embodiments thereof.

### [Subject]

In the present invention, a "subject" refers to a person from whom feces, which is a sample for the diagnostic assistance method and diagnostic method of the present invention, is derived, and refers to a subject for performing the diagnostic assistance method and diagnostic method. Examples of the subject include humans or non-human mammals (mice, rats, guinea pigs, hamsters, rabbits, monkeys, dogs, cats, horses, cows, sheep, goats, pigs, etc.), and humans are preferred. The subject according to the present invention is not particularly limited, and may be a healthy subject not afflicted with a gastrointestinal disease, or a person who is afflicted with a gastrointestinal disease but has not developed symptoms. The subject may also be a patient afflicted with a gastrointestinal disease, or may be a known patient who is already afflicted with a gastrointestinal disease, a patient who was afflicted in the past, or the like, for the purpose of follow-up observation of the gastrointestinal disease or prognostic observation of treatment.

### [Feces]

The sample for the diagnostic assistance method and diagnostic method of the present invention, the screening method of the present invention, and the detection method of the present invention is "feces." The feces are not particularly limited as long as the fecal exosomes and gastrointestinal disease-related proteins described below can be present. Generally, examples include feces collected from the subject, etc., a human or a non-human mammal, and may be one collected directly from the large intestine of a human or a non-human mammal by endoscopy or the like. The feces may be one suspended in a suspension solution as necessary, and examples of the suspension solution in this case include buffer solutions such as phosphate buffer, Tris buffer, Good's buffer, borate buffer, acetate buffer, citrate buffer, glycine buffer, succinate buffer, and phthalate buffer. Furthermore, the feces may be one to which a pH adjuster, stabilizer, preservative, antiseptic, surfactant, or the like has been added, or one that has been frozen, etc., as necessary.

### [Fecal Exosomes]

In the present invention, "fecal exosomes" are exosomes contained in the feces or separated from the feces. In contrast, in this specification, the entire feces before the exosome separation (that is, the sample itself) is sometimes referred to as "total feces." Exosomes generally refer to intracellular vesicles formed in intracellular multivesicular endosomes (MVEs) by the inward budding of the MVE membrane, but the "fecal exosomes" in the present invention refer to vesicles among extracellular vesicles derived from a human or non-human mammal (preferably a human) from which the feces were collected, that are present in the feces, have a particle size of 30 to 300 nm, and have at least one exosome marker protein selected from the group consisting of Alix, CD9, CD63, CD81, and Tsg101 on the surface or inside. Furthermore, the "fecal exosomes" according to the present invention are preferably vesicles contained in a fraction in which the exosome marker protein is detected, preferably a fraction with an iodixanol concentration of 30 to 45%, among fractions obtained by density gradient centrifugation of a suspension of a precipitate obtained by a centrifugal force of 150,000 × g (for example, a phosphate buffer suspension) from a supernatant of a suspension of the feces (for example, a phosphate buffer suspension) (for example, a supernatant obtained by centrifugation at a centrifugal force of 3,000 to 4,000 × g for 10 to 90 minutes, 3 to 4 times), using 5 to 40% iodixanol as a separation medium and a centrifugal force of 100,000 × g.

The particle size can be measured, for example, by nanoparticle tracking analysis (for example, analysis by NanoSight (manufactured by Malvern Panalytical) as described in the Examples) which measures the particle size of particles in a particle liquid from the velocity of their Brownian motion, and the particle size of the "fecal exosomes" according to the present invention is more preferably 100 to 300 nm.

The fraction in which the exosome marker protein is detected may be a fraction in which one type of the exosome marker proteins is detected, but it is more preferably a fraction in which two or more types are detected. Examples of the method for detecting these exosome marker proteins include a method of forming an immune complex between each exosome marker protein and a labeled body in which a labeling substance is bound to an antibody against it (detection antibody), and detecting a signal corresponding to the labeling substance. In the present invention, "signal" includes color development (coloration), quenching, reflected light, luminescence, fluorescence, radiation from a radioactive isotope, and the like, depending on the labeling substance, and includes not only those that can be confirmed with the naked eye but also those that can be confirmed by a detection method or device corresponding to the type of signal.

In the present invention, the "antibody" may be a polyclonal antibody or a monoclonal antibody. Furthermore, in the present invention, "antibody" includes, in addition to complete antibodies, antibody fragments (for example, Fab, Fab', F(ab')₂, Fv, disulfide-linked Fv, single-chain Fv (scFv), sc(Fv)₂, Diabody) and polymers thereof. Also, in the present invention, "antibody" includes all classes (isotypes) and subclasses of immunoglobulins, and two or more types may be appropriately combined, but IgG is preferred. Furthermore, the origin of such an antibody is not particularly limited, and may be a human-derived antibody, a non-human animal-derived antibody (for example, rabbit antibody, mouse antibody, rat antibody, goat antibody, sheep antibody, camel antibody), a chimeric antibody, or a humanized antibody. Such an antibody can be appropriately prepared by a conventionally known method, and a commercially available product may also be used as appropriate.

As the "labeling substance," those used as labeling substances in conventionally known immunological detection methods or methods analogous thereto can be used without particular limitation. Examples of such labeling substances include enzymes; luminescent substances such as acridinium derivatives; fluorescent substances such as europium; fluorescent proteins such as allophycocyanin (APC) and phycoerythrin (R-PE); radioactive substances such as ¹²⁵I; fluorescent dyes such as fluorescein isothiocyanate (FITC) and rhodamine isothiocyanate (RITC); particles such as latex particles and gold particles; dinitrophenyl (DNP); and digoxigenin (DIG), and one of these or a combination of two or more may be used. For example, when an enzyme is used as the labeling substance, by adding a colorimetric substrate, a fluorescent substrate, a chemiluminescent substrate, or the like as a substrate, detection of various signals corresponding to the substrate can be performed. Examples of the enzyme include, but are not limited to, peroxidase (POD: horseradish peroxidase (HRP), etc.), alkaline phosphatase (ALP), β-galactosidase (β-gal), glucose oxidase, and luciferase.

For the binding between the labeling substance and the antibody, a conventionally known method or a method analogous thereto can be appropriately employed; the antibody and the labeling substance may be directly bound by an active group or the like, may be indirectly bound via an oligopeptide, a linker, or the like, or a biotin-avidin system (for example, biotinylating an antibody, reacting it with an avidin-conjugated labeling substance, and binding the labeling substance to the antibody using the interaction between biotin and avidin) may be used. The labeling substance may be bound to the antibody, but it may also be indirectly bound to the antibody using a secondary antibody or the like to which the labeling substance is bound, without binding the labeling substance to the antibody. Here, a "secondary antibody" is an antibody that exhibits reactivity to an antibody that directly binds to a substance to be detected (primary antibody). In place of a secondary antibody, protein G, protein A, or the like to which a labeling substance is bound may be used.

### [Gastrointestinal Disease-Related Protein]

In the present invention, examples of the "gastrointestinal tract" include the stomach, small intestine (duodenum, jejunum, ileum), large intestine (cecum, appendix, colon, rectum), and anus. Among these, as the "gastrointestinal tract" according to the present invention, at least one selected from the group consisting of the small intestine (duodenum, jejunum, ileum) and the large intestine (cecum, appendix, colon, rectum) is preferred.

In the present invention, examples of "gastrointestinal disease" include diseases of the gastrointestinal tract, and are not particularly limited, but for example, gastric diseases such as gastric cancer and gastritis; small intestine diseases such as small intestine cancer; and large intestine diseases such as colorectal cancer (colon cancer, rectal cancer, colorectal cancer, etc.), colitis, and Crohn's disease can be mentioned, and one of these or a combination of two or more may be used. Among these, as the gastrointestinal disease according to the present invention, an intestinal disease (small intestine disease, large intestine disease) is preferred, more preferably a large intestine disease, and still more preferably colorectal cancer.

In the present invention, the "gastrointestinal disease-related protein" is not particularly limited as long as it is a protein that has already been reported to be related to the gastrointestinal disease or may be related to a gastrointestinal disease; examples include proteins derived from the gastrointestinal tract whose expression level increases or decreases in relation to a gastrointestinal disease, and proteins derived from gastrointestinal cancer, and one type or a combination of two or more types may be used.

More specifically, examples of the gastrointestinal disease-related protein according to the present invention include the proteins described in Tables 10 to 12 below, that is, ZNF256, ZMPSTE24, YWHAH, YWHAG, WDR1, VILL, VASP, VAMP8, USP5, UGDH, UBE2D3, TUBB4B, TSPAN6, TPP1, TPM4, TPM3, TPM1, TMEM30A, TC2N, SYPL1, SUSD2, SURF4, STOM, ST6GALNAC1, ST13, SQOR, SPTBN1, SPPL2A, SMPD3, SLC9A3, SLC6A14, SLC46A1, SLC44A1, SLC36A1, SLC2A5, SLC25A5, SLC25A4, SLC16A1, SLC15A4, SLC15A1, SERPINF2, SERPINA7, SDHB, SDHA, SCARB2, SAFB2, S100A11, RPS7, RPS6KA1, RPS3, RPS23, RPS16, RPS10, RPL35, RPL30, RNPEP, RNF128, RNASE3, RNASE1, RHPN2, RAB3D, RAB32, RAB2A, RAB21, RAB14, PTPN6, PSMB8, PSMB1, PSMA1, PRKG2, PRKCD, PRKACA, PRDX6, PRDX5, PRDX2, PRDX1, PPP1CB, PNP, PLEC, PLA2G2A, PLA2G10, PKM, PICALM, PGK1, PGD, PFN1, PCYOX1, PCNT, PCBP1, PAFAH1B1, P2RX4, OTUB1, NPEPPS, NIBAN2, NEDD4L, NCSTN, MYOF, MYO1E, MYO1C, MYO1B, MYH9, MYH14, MUC4, MUC3A, MT-CYB, MIS12, MIF, MGST3, MGLL, MEP1A, MAOB, MAOA, LYN, LSM2, LGALS4, LGALS3BP, LDHA, LAMP1, ITGA6, ITGA3, ITGA1, IGLV7-46, IGKV1-39, IGHM, IGHG4, IGHG2, IDH1, HTATIP2, HSPA5, HSP90AB1, HSD17B4, HNRNPA2B1, HM13, HLA-DMA, HARS1, H2BC12, H1-4, GSTP1, GSTA1, GSDME, GRN, GPI, GPD2, GPA33, GP2, GNAI2, GMDS, GHITM, GFM2, GDPD3, GAPDH, FUT3, FLOT2, FLOT1, F11R, EVC2, ETHE1, ESD, ERLIN2, EPCAM, ENPP4, ENOl, EIF5A, EIF4A1, EEF2, EBP, DSTN, DNPEP, DLD, DDOST, CYFIP1, CSRP1, CSNK1A1, CPNE3, CORO1B, CNDP2, CLTC, CLIC1, CHP2, CHMP2A, CEACAM5, CEACAM1, CD151, CASQ1, CAPZB, CAPZA1, CAPN1, CAP1, C9, BLVRB, B3GNT7, B2M, ATP9A, ATP1B3, ATP1B1, ATP1A1, ASAH1, ARPC5, ARPC2, ARL1, ARHGDIA, AP2M1, AP2A1, AP1B1, ANXA4, ANXA3, ANO6, ALDOA, ALDH3B1, AGR3, AGR2, ACTR3, ACTR2, ACTN4, ACAA1, and ABRACL.

Furthermore, examples of the gastrointestinal disease-related protein according to the present invention also include the proteins described in Tables 13 to 15 below, that is, YARS1, XRCC6, XRCC5, XPO1, WDR44, VPS35, VPS29, VPS26A, VKORC1L1, VBP1, VAMP7, USP15, USP14, USO1, UQCRC2, UQCRC1, UPF1, UGGT1, UBA1, TUFM, TPP2, TPMT, TOP2B, TMEM33, TMEM205, TMED9, TMED7, TMED5, TMED4, TMED2, TMED10, TM9SF4, TM9SF3, TM9SF2, TM4SF20, TKT, THEMIS2, TEKT2, TBXAS1, TARS1, TARDBP, SYNGR2, SYK, SUCLG2, SUCLG1, SUCLA2, STXBP6, STIP1, STAT3, STAT1, SSR4, SSR1, SRSF3, SRM, SPCS3, SPAST, SORL1, SORD, SOD2, SNX33, SNRPA1, SNRNP70, SND1, SLK, SLC6A6, SLC3A2, SLC35B2, SLC2A3, SLC2A1, SLC25A11, SLC16A3, SLC12A9, SLC11A2, SIRPA, SIL1, SIGMAR1, SHMT2, SHMT1, SF3B3, SERPINB5, SERPINA6, SEPTIN7, SELENOP, SEC31A, SEC24D, SEC24C, SEC23B, SEC23A, SACM1L, RUVBL2, RRM2B, RRAGC, RRAGB, RPS9, RPS4X, RPS3A, RPS24, RPS18, RPS17, RPS14, RPS13, RPN1, RPLPO, RPL7, RPL6, RPL5, RPL4, RPL3, RPL28, RPL27A, RPL24, RPL21, RPL18A, RPL18, RPL17, RPL14, RPL13A, RPL13, RPL12, RPL10A, RNF149, REEP6, RCC2, RBBP4, RARS1, RACK1, RABGGTA, QARS1, PYGB, PTPRE, PTK2B, PTGES3, PSMF1, PSMD7, PSMD6, PSMD3, PSMD2, PSMD13, PSMD12, PSMD1, PSMC5, PSMC3, PSMC2, PRXL2A, PRPS1, PRPF19, PRMT1, PRKDC, PRKCB, PPP2CB, PPIB, POR, PON1, POLR2H, PLTP, PLP2, PLEKHA1, PLCG2, PITPNA, PIP4P2, PHLDA3, PHB2, PGAP6, PFKP, PDLIM1, PCK2, PCCA, PCBP2, PC, PARP4, PAK4, PAICS, PABPC1, PAAF1, PA2G4, OGDH, OCIAD2, OCIAD1, OAT, OAF, NXF1, NT5C2, NSDHL, NQO1, NPC1, NOSTRIN, NNT, NMT1, NIPSNAP2, NIBAN1, NDUFV1, NDUFB10, NCKAP1L, NARS1, MVD, MT-CO2, MOGS, MOB1B, MICAL1, MGST2, MGST1, MFGE8, MCCC2, MAT2A, MAPK14, MAPK1, MAN1A1, MACC1, M6PR, LYPLAL1, LTA4H, LNPEP, LBR, LARS1, KDELR2, KDELR1, KARS1, JAKMIP3, ITGAV, ITGA2, IQGAP2, IMPDH2, ILF2, IL18R1, IGKV1-12, IDH3B, IDH3A, IDH2, IDE, IARS1, HTT, HSPH1, HSPA9, HSPA4, HSP90B1, HSDL2, HSD17B11, HSD17B10, HSD11B2, HNRNPUL2, HNRNPU, HNRNPK, HNRNPC, HMGCS2, HMGB1, HLA-B, HKDC1, HK2, HK1, HIBCH, HCK, HADHB, HADHA, HADH, H1-5, H1-0, GYS1, GSPT1, GSK3A, GRTP1, GRK2, GPLD1, GPD1, GOT2, GOLGB1, GNPNAT1, GMPPB, GDI1, GCLM, GCLC, GBE1, GATD3B, GART, GARS1, GANAB, GALNT2, GALNT1, G6PD, FGR, FETUB, FCN3, FBXO7, FBXO6, FASN, FARSB, F3, ETFDH, ETF1, ERO1A, ERAP2, ERAP1, ENTPD1, EML4, EIF3L, EIF3I, EIF3D, EIF3CL, EIF3B, EIF2S3, EIF2S1, EFR3A, EEF1G, DYNC1H1, DPYSL2, DOP1B, DNM2, DNAJC13, DNAJB11, DLAT, DHX9, DHRS9, DHRS7, DDX1, DDC, DARS1, DAD1, CYFIP2, CYB5R3, CYB5B, CSK, CRYZ, CRYL1, CPT2, CPT1A, CPB2, COX5B, CORO7, CORO1C, COPG1, COPB2, COPA, CMPK1, CLPX, CLINT1, CLDN7, CHST5, CEMIP2, CDCP1, CCT8, CCT7, CCT6A, CCT5, CCT4, CCT3, CCT2, CASP6, C8G, C8B, C7, BUB3, BST2, BSG, BPHL, BLVRA, BAX, BACE1, B3GNT8, B3GNT3, ATP8A1, ATP6V1H, ATP6V1G1, ATP6V1E1, ATP6V1A, ATP6V0D1, ATP6V0A1, ATP5PO, ATP5PB, ATP5MG, ATP5MF, ATP5F1A, ATP2A2, ATP11B, ATIC, ATG3, ASL, ARPC3, ARPC1B, ARHGAP1, ARCN1, APRT, APPL2, APPL1, APMAP, APIP, APEX1, AP1S1, AP1M1, AP1G1, ALOX15, ALDH9A1, ALDH16A1, AKR1C3, AIMP1, AIFM1, AHSA1, AGPS, AGO2, ADSS2, ADGRG3, ADGRE5, ADAM10, ACSL5, ACSL1, ACOT7, ACOT11, ACLY, ACADS, ACADM, ACAA2, ABRAXAS2, ABCG8, and ABCF1.

Furthermore, examples of the gastrointestinal disease-related protein according to the present invention also include the proteins described in Table 16 below, that is, LCP1, ANXA1, OLFM4, FBLN1, COL6A1, TGFBI, and THBS1.

In addition, other examples of the gastrointestinal disease-related protein according to the present invention include SLC25A3, ACADM, ACOT7, ACOX1, ACSL5, ADGRE5, ADGRG7, ADH6, AGR2, AHCYL1, AK1, AKAP7, AKR1A1, AKR1C3, ALDH9A1, ALDOA, ANPEP, ANXA13, ANXA2, ANXA3, ANXA7, APIP, APOA4, APOB, APPL2, ARHGDIA, ARPC2, ATIC, ATP10B, ATP11B, ATP5F1A, ATP8B1, BAIAP2L2, C2, C8G, CALM1, CALML4, CAP1, CCT7, CD151, CD177, CD300LF, CD59, CD82, CEACAM1, CEACAM5, CEACAM7, CEL, CHP1, CHP2, CKB, CLCA1, CLDN3, CLDN7, CLTC, CLU, CNDP2, COPA, CORO7, CSNK1G2, CYB5B, CYBRD1, DDAH1, DDOST, DDX3X, DEFA5, DHRS9, DMBT1, DPEP1, EEF1A1, EIF2S3, EIF3L, ENOl, ENPP7, EPCAM, EPHA2, EPS8L3, EZR, FABP1, FAM151A, FBP1, FBP2, FERMT1, FOLH1, FTH1, FUT3, GAPDH, GBA1, GDA, GFM2, GIPC1, GIPC2, GNPNAT1, GPA33, GPRC5A, GSTP1, H1-0, HADHB, HLA-DRA, HLA-DRB1, HMGB1, HNRNPA2B1, HNRNPK, HNRNPU, HPGD, HSD17B11, HSD17B2, HSP90AB1, HSPA1A, HSPA4, HSPA5, HSPA8, HSPA9, IDH2, ILF2, IMPDH2, ITLN1, ITLN2, KCNAB2, KIF20B, KLKB1, KRAS, LASP1, LCT, LGALS3BP, LGALS4, LGALS9, LGALS9B, LNX2, LPCAT3, LSR, LYZ, MEP1A, MEP1B, MINK1, MS4A12, MTTP, MUC12, MUC13, MUC17, MUC3A, MUC4, MYH14, MYO15B, MYO1A, MYO1D, NAALADL1, NARS1, NCF2, NCKAP1L, NDUFV1, NIPSNAP2, NOS2, NOSTRIN, NOX1, NOXO1, NQO1, OCLN, OTUB1, PA2G4, PAFAH1B1, PARP4, PDCD6, PDCD6IP, PDZK1, PFKL, PFN1, PGA4, PGK1, PKM, PLEC, PLEKHA1, PLS1, PLSCR1, PLTP, POR, PPIA, PPP1CC, PPP2R1A, PROM1, PRPS1, PRSS8, PSMF1, PTPRB, PTPRJ, PYGB, RAB10, RAB1A, RAB21, RAB22A, RAB25, RAC1, RAN, RBP2, REG4, RHOA, RNASE3, RPL13A, RPL18, RPL21, RPL24, RPL27A, RPL28, RPL3, RPL35A, RPL5, RPLPO, RPS10, RPS15A, RPS18, RPS2, RPS3, RPS6KA1, RPS9, RUVBL2, SAFB2, SDHA, SERINC2, SERPINA4, SERPINB5, SI, SLC15A1, SLC16A1, SLC23A1, SLC25A3, SLC25A5, SLC26A3, SLC28A2, SLC44A4, SLC9A3, SLPI, SMPDL3B, SOD2, SPINT1, SRI, STK25, STX3, STXBP2, SULT1A1, SYTL2, TAGLN2, TCP1, TFF3, TM4SF20, TM9SF2, TM9SF3, TMBIM1, TMC4, TMED10, TMIGD1, TPM1, TSPAN1, TSPAN8, TUT1, UBB, UBE2V1, UPP1, USH1C, VDAC2, VIL1, VPS26A, VPS35, VPS37B, VTA1, WDR1, XDH, XPNPEP2, YWHAB, YWHAG, YWHAZ, ZG16, AHCYL1, CEACAM7, GUCY2C, HSPA8, RAB1A, SLC44A4, SULT1A1, and VIL1.

### <Method for assisting diagnosis of gastrointestinal disease, Diagnostic method for gastrointestinal disease>

The present invention provides a method for assisting diagnosis of a gastrointestinal disease (in this specification, sometimes referred to as "the diagnostic assistance method of the present invention"), comprising:
a step of separating fecal exosomes from feces of a subject, and
a step of detecting a gastrointestinal disease-related protein from the separated fecal exosomes.

The present invention also provides a diagnostic method for a gastrointestinal disease (in this specification, sometimes referred to as "the diagnostic method of the present invention"), comprising:
a step of separating fecal exosomes from feces of a subject, and
a step of detecting a gastrointestinal disease-related protein from the separated fecal exosomes.

The present inventors found that because gastrointestinal disease-related proteins are significantly concentrated in fecal exosomes, by first separating fecal exosomes from feces and then detecting the gastrointestinal disease-related protein from the separated fecal exosomes, the detection of the gastrointestinal disease-related protein can be performed with high efficiency and high precision using only the simple technique of separating fecal exosomes from the feces.

### [Separation Step]

The diagnostic assistance method and diagnostic method of the present invention include a step of separating the fecal exosomes from the feces of the subject (in the diagnostic assistance method and diagnostic method of the present invention, "separation step") .

The method for separating fecal exosomes from the feces is not particularly limited as long as it is a method capable of separating the fecal exosomes according to the present invention described above, and a conventionally known method or a method analogous thereto can be appropriately employed. Examples of such a separation method include, in addition to the density gradient centrifugation method described above, density gradient centrifugation methods using a separation medium other than iodixanol (for example, a method using sucrose: bio-protocol 10(08):e3584, DOI:10.21769/BioProtoc.3584, 2020); ultracentrifugation; immunological methods of capturing with an antibody against the exosome marker protein (for example, immunoprecipitation with beads on which the antibody is immobilized); elution methods with a size exclusion chromatography column; precipitation methods using a polymer such as polyethylene glycol; and size exclusion methods with an ultrafiltration membrane, and two or more of these may be combined. As the method for separating the fecal exosomes according to the present invention, among these, the density gradient centrifugation method using iodixanol as a separation medium described above is preferred from the viewpoint of ease and the tendency to be able to separate the fecal exosomes from feces with high purity. A method of further performing density gradient centrifugation on a crude product obtained by separating with a separation method other than density gradient centrifugation (for example, centrifugation, filtration, etc.) is also preferred, and this can further improve the purity of the separated fecal exosomes. As a method for separating fecal exosomes from feces in which many contaminants are mixed, the purity of the fecal exosomes can be further increased by combining purification methods in this way.

After the separation step, for example, the presence and purity of the separated fecal exosomes may be confirmed by further performing confirmation of expression of an exosome marker protein by Western blotting or the detection method described above, particle size measurement, observation using an electron microscope, or a combination thereof. Therefore, the diagnostic assistance method and diagnostic method of the present invention may further include a confirmation step for confirming the presence and purity of such fecal exosomes.

### [Detection Step]

The diagnostic assistance method and diagnostic method of the present invention include a step of detecting the gastrointestinal disease-related protein from the fecal exosomes separated in the separation step (in the diagnostic assistance method and diagnostic method of the present invention, "detection step").

As the gastrointestinal disease-related protein to be detected in the diagnostic assistance method and diagnostic method of the present invention, preferably, the gastrointestinal disease-related proteins described above are mentioned, and one of these or two or more thereof may be used. Furthermore, as the gastrointestinal disease-related protein to be detected in the diagnostic assistance method and diagnostic method of the present invention, it is preferably a protein that is concentrated within fecal exosomes, and for which the content ratio relative to the total amount of fecal exosomes is greater than the content ratio relative to the total amount of the feces (total feces), or one that is detected only from the fecal exosomes and is substantially not detected from the feces. More specifically, the gastrointestinal disease-related protein according to the diagnostic assistance method and diagnostic method of the present invention is a gastrointestinal disease-related protein that satisfies any one of the following conditions (a) and (b):
(a) a content ratio relative to a total amount of the fecal exosomes is 1.5 times or more a content ratio relative to a total amount of the feces,
(b) the protein is detected only from the separated fecal exosomes and is substantially not detected from the feces.

Here, the content ratio of the gastrointestinal disease-related protein relative to the total amount of fecal exosomes may be 1.5 times or more the content ratio relative to the total amount of the feces (total feces), but it is more preferably 2 times or more. The upper limit of the ratio is not particularly limited. "Substantially not detected" from feces means that the content in the total amount of the feces is below the detection limit. The method for detecting a gastrointestinal disease-related protein from feces or fecal exosomes and the method for quantifying a gastrointestinal disease-related protein are as described below. For example, when the immunological detection method described below is used as the detection method according to the present invention, each content ratio can be determined from the protein amount quantified based on the measured value of the signal and the sample amount provided for the measurement (that is, the total amount of total feces or the total amount of fecal exosomes, the same applies hereinafter), and "substantially not detected" means that when total feces and fecal exosomes are provided for the same measurement with the same sample amount, no signal is detected in the total feces by the detection method corresponding to the labeling substance described below, or the signal is at or below the detection limit (usually 3 times the standard deviation). For example, when MS/MS analysis or LC/MS/MS analysis described below is used as the detection method according to the present invention, each content ratio can be determined from the absolute quantified value quantified based on the analysis result as the content amount and the sample amount provided for the measurement, but in the case of comparing the content ratio relative to the total amount of the fecal exosomes and the content ratio relative to the total amount of total feces, their abundance ratio (ratio of relative quantified values) may be substituted for the ratio of content ratios. When MS/MS analysis or LC/MS/MS analysis is used, "substantially not detected" means that when total feces and fecal exosomes are provided for measurement with the same sample amount, no spectrum is observed in the total feces, or the spectrum is at or below the detection limit (usually 3 times the standard deviation).

Examples of such a gastrointestinal disease-related protein include the gastrointestinal disease-related proteins described above, but among these, at least one selected from the group consisting of the proteins described in Tables 10 to 15 above is further preferred from the viewpoint of being significantly concentrated in fecal exosomes and particularly concentrated in fecal exosomes of a gastrointestinal disease patient (preferably a colorectal cancer patient).

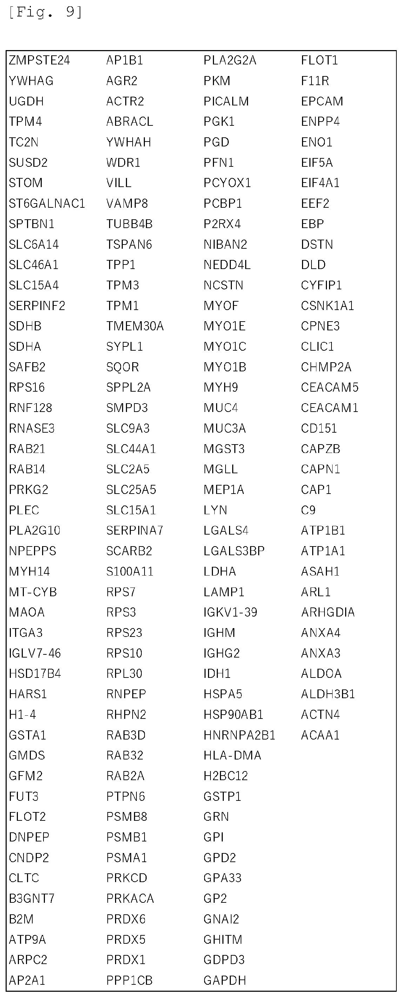

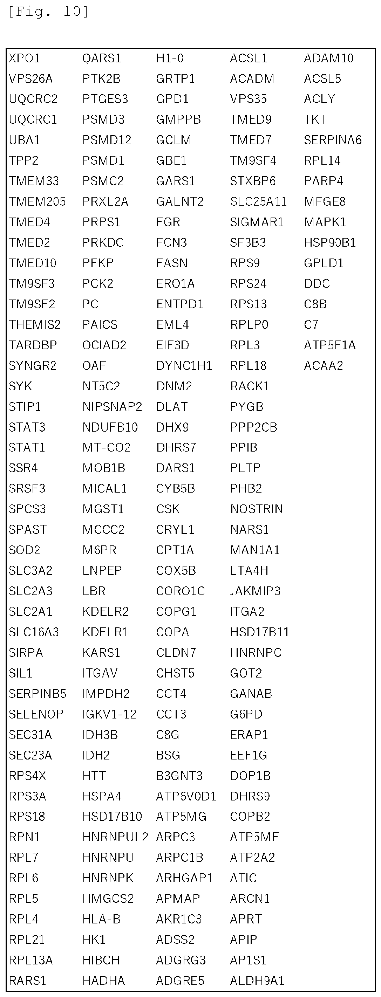

Among these, at least one selected from the group consisting of the proteins described in Fig. 9, that is, ZMPSTE24, YWHAG, UGDH, TPM4, TC2N, SUSD2, STOM, ST6GALNAC1, SPTBN1, SLC6A14, SLC46A1, SLC15A4, SERPINF2, SDHB, SDHA, SAFB2, RPS16, RNF128, RNASE3, RAB21, RAB14, PRKG2, PLEC, PLA2G10, NPEPPS, MYH14, MT-CYB, MAOA, ITGA3, IGLV7-46, HSD17B4, HARS1, H1-4, GSTA1, GMDS, GFM2, FUT3, FLOT2, DNPEP, CNDP2, CLTC, B3GNT7, B2M, ATP9A, ARPC2, AP2A1, AP1B1, AGR2, ACTR2, ABRACL, YWHAH, WDR1, VILL, VAMP8, TUBB4B, TSPAN6, TPP1, TPM3, TPM1, TMEM30A, SYPL1, SQOR, SPPL2A, SMPD3, SLC9A3, SLC44A1, SLC2A5, SLC25A5, SLC15A1, SERPINA7, SCARB2, S100A11, RPS7, RPS3, RPS23, RPS10, RPL30, RNPEP, RHPN2, RAB3D, RAB32, RAB2A, PTPN6, PSMB8, PSMB1, PSMA1, PRKCD, PRKACA, PRDX6, PRDX5, PRDX1, PPP1CB, PLA2G2A, PKM, PICALM, PGK1, PGD, PFN1, PCYOX1, PCBP1, P2RX4, NIBAN2, NEDD4L, NCSTN, MYOF, MYO1E, MYO1C, MYO1B, MYH9, MUC4, MUC3A, MGST3, MGLL, MEP1A, LYN, LGALS4, LGALS3BP, LDHA, LAMP1, IGKV1-39, IGHM, IGHG2, IDH1, HSPA5, HSP90AB1, HNRNPA2B1, HLA-DMA, H2BC12, GSTP1, GRN, GPI, GPD2, GPA33, GP2, GNAI2, GHITM, GDPD3, GAPDH, FLOT1, F11R, EPCAM, ENPP4, ENO1, EIF5A, EIF4A1, EEF2, EBP, DSTN, DLD, CYFIP1, CSNK1A1, CPNE3, CLIC1, CHMP2A, CEACAM5, CEACAM1, CD151, CAPZB, CAPN1, CAP1, C9, ATP1B1, ATP1A1, ASAH1, ARL1, ARHGDIA, ANXA4, ANXA3, ALDOA, ALDH3B1, ACTN4, and ACAA1; the proteins described in Fig. 10, that is, XPO1, VPS26A, UQCRC2, UQCRC1, UBA1, TPP2, TMEM33, TMEM205, TMED4, TMED2, TMED10, TM9SF3, TM9SF2, THEMIS2, TARDBP, SYNGR2, SYK, STIP1, STAT3, STAT1, SSR4, SRSF3, SPCS3, SPAST, SOD2, SLC3A2, SLC2A3, SLC2A1, SLC16A3, SIRPA, SIL1, SERPINB5, SELENOP, SEC31A, SEC23A, RPS4X, RPS3A, RPS18, RPN1, RPL7, RPL6, RPL5, RPL4, RPL21, RPL13A, RARS1, QARS1, PTK2B, PTGES3, PSMD3, PSMD12, PSMD1, PSMC2, PRXL2A, PRPS1, PRKDC, PFKP, PCK2, PC, PAICS, OCIAD2, OAF, NT5C2, NIPSNAP2, NDUFB10, MT-CO2, MOB1B, MICAL1, MGST1, MCCC2, M6PR, LNPEP, LBR, KDELR2, KDELR1, KARS1, ITGAV, IMPDH2, IGKV1-12, IDH3B, IDH2, HTT, HSPA4, HSD17B10, HNRNPUL2, HNRNPU, HNRNPK, HMGCS2, HLA-B, HK1, HIBCH, HADHA, H1-0, GRTP1, GPD1, GMPPB, GCLM, GBE1, GARS1, GALNT2, FGR, FCN3, FASN, ERO1A, ENTPD1, EML4, EIF3D, DYNC1H1, DNM2, DLAT, DHX9, DHRS7, DARS1, CYB5B, CSK, CRYL1, CPT1A, COX5B, CORO1C, COPG1, COPA, CLDN7, CHST5, CCT4, CCT3, C8G, BSG, B3GNT3, ATP6V0D1, ATP5MG, ARPC3, ARPC1B, ARHGAP1, APMAP, AKR1C3, ADSS2, ADGRG3, ADGRE5, ACSL1, ACADM, VPS35, TMED9, TMED7, TM9SF4, STXBP6, SLC25A11, SIGMAR1, SF3B3, RPS9, RPS24, RPS13, RPLPO, RPL3, RPL18, RACK1, PYGB, PPP2CB, PPIB, PLTP, PHB2, NOSTRIN, NARS1, MAN1A1, LTA4H, JAKMIP3, ITGA2, HSD17B11, HNRNPC, GOT2, GANAB, G6PD, ERAP1, EEF1G, DOP1B, DHRS9, COPB2, ATP5MF, ATP2A2, ATIC, ARCN1, APRT, APIP, AP1S1, ALDH9A1, ADAM10, ACSL5, ACLY, TKT, SERPINA6, RPL14, PARP4, MFGE8, MAPK1, HSP90B1, GPLD1, DDC, C8B, C7, ATP5F1A, and ACAA2; and the proteins described in Table 16, that is, LCP1, ANXA1, OLFM4, FBLN1, COL6A1, TGFBI, and THBS1, is preferred.

The present invention also provides, as the diagnostic assistance method and diagnostic method of the present invention, a method comprising the separation step and the detection step, wherein the gastrointestinal disease-related protein is selected from the group consisting of the proteins described in Fig. 9 above, the proteins described in Fig. 10, and the proteins described in Table 16.

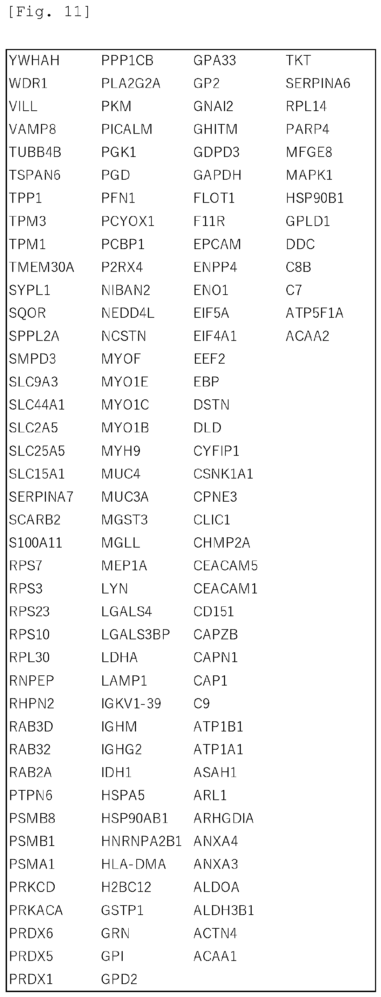

Among the above, as the gastrointestinal disease-related protein detected in each of the diagnostic assistance method and diagnostic method of the present invention, particularly preferred is at least one selected from the group consisting of the proteins described in Fig. 11, that is, YWHAH, WDR1, VILL, VAMP8, TUBB4B, TSPAN6, TPP1, TPM3, TPM1, TMEM30A, SYPL1, SQOR, SPPL2A, SMPD3, SLC9A3, SLC44A1, SLC2A5, SLC25A5, SLC15A1, SERPINA7, SCARB2, S100A11, RPS7, RPS3, RPS23, RPS10, RPL30, RNPEP, RHPN2, RAB3D, RAB32, RAB2A, PTPN6, PSMB8, PSMB1, PSMA1, PRKCD, PRKACA, PRDX6, PRDX5, PRDX1, PPP1CB, PLA2G2A, PKM, PICALM, PGK1, PGD, PFN1, PCYOX1, PCBP1, P2RX4, NIBAN2, NEDD4L, NCSTN, MYOF, MYO1E, MYO1C, MYO1B, MYH9, MUC4, MUC3A, MGST3, MGLL, MEP1A, LYN, LGALS4, LGALS3BP, LDHA, LAMP1, IGKV1-39, IGHM, IGHG2, IDH1, HSPA5, HSP90AB1, HNRNPA2B1, HLA-DMA, H2BC12, GSTP1, GRN, GPI, GPD2, GPA33, GP2, GNAI2, GHITM, GDPD3, GAPDH, FLOT1, F11R, EPCAM, ENPP4, ENO1, EIF5A, EIF4A1, EEF2, EBP, DSTN, DLD, CYFIP1, CSNK1A1, CPNE3, CLIC1, CHMP2A, CEACAM5, CEACAM1, CD151, CAPZB, CAPN1, CAP1, C9, ATP1B1, ATP1A1, ASAH1, ARL1, ARHGDIA, ANXA4, ANXA3, ALDOA, ALDH3B1, ACTN4, ACAA1, TKT, SERPINA6, RPL14, PARP4, MFGE8, MAPK1, HSP90B1, GPLD1, DDC, C8B, C7, ATP5F1A, and ACAA2; and OLFM4, LCP1, and ANXA1.

The gastrointestinal disease-related protein is still more preferably at least one selected from the group consisting of OLFM4, LGALS3BP, LAMP1, ATP5F1A, DLD, ENPP4, MAPK1, PCYOX1, SMPD3, VILL, and RPS7.

The method for detecting the gastrointestinal disease-related protein (in this specification, sometimes simply referred to as "detection method") is not particularly limited as long as it is a method capable of detecting it, depending on the type of gastrointestinal disease-related protein to be detected, and a conventionally known method or a method analogous thereto can be appropriately employed. Examples thereof include immunological detection methods using an antibody against the gastrointestinal disease-related protein; MS/MS analysis; LC/MS/MS analysis; and Western blotting, and two or more methods may be appropriately combined. As the detection method according to the present invention, among these, an immunological detection method is preferred.

As the immunological detection method, a conventionally known method, a method analogous thereto, or a combination thereof can be appropriately employed. Examples of such an immunological detection method include a method of forming an immune complex between each gastrointestinal disease-related protein and a labeled body in which a labeling substance is bound to an antibody against it (detection antibody), and detecting a signal corresponding to the labeling substance; examples thereof include EIA (enzyme immunoassay) using an enzyme as a labeling substance, ELISA, which is a mode of EIA, CLIA (chemiluminescence immunoassay) using a chemiluminescent compound as a labeling substance, IRMA or RIA (radioimmunoassay) using a radioisotope as a labeling substance, and combinations thereof (for example, CLEIA (chemiluminescent enzyme immunoassay), BLEIA (bioluminescent enzyme immunoassay)); immunochromatography; and immunoagglutination methods (latex agglutination, gold colloid agglutination, etc.) by immunonephelometry, immunoturbidimetry, etc., but are not limited to these. The detection method according to the present invention may be a non-competitive detection method or a competitive detection method.

As the detection method according to the present invention, sandwich methods such as ELISA, CLIA, IRMA, CLEIA, and BLEIA are preferred from the viewpoint that they tend to have higher sensitivity. In a sandwich method, a substance to be detected (in the present invention, a gastrointestinal disease-related protein) is captured by a capture antibody (capture body) immobilized on a solid phase, and it is recognized by a detection antibody (labeled body) bound to a labeling substance (labeled) to form a complex of capture antibody-substance to be detected-detection antibody, and after B/F separation (washing), the complex is detected by a signal corresponding to the type of labeling substance. Such sandwich methods include the forward sandwich method, which is a two-step method (a method of sequentially reacting a capture antibody and a substance to be detected, and reacting a substance to be detected bound to a capture antibody and a detection antibody), the reverse sandwich method (a method of reacting a detection antibody and a substance to be detected in advance, and reacting the generated complex with a capture antibody), and the one-step method (a method of reacting a substance to be detected, a capture antibody, and a detection antibody simultaneously in one step), any of which can be employed. Alternatively, like in an immunochromatography method, the substance to be detected may be recognized by a detection antibody, and while performing B/F separation, the substance to be detected may be captured by a capture antibody, and a signal corresponding to the type of labeling substance may be detected.

The capture antibody and the detection antibody are antibodies against the substance to be detected, and such antibodies are as described above and can be appropriately selected according to the gastrointestinal disease-related protein that is the substance to be detected. The labeling substance, the binding of the detection antibody and the labeling substance, and the signal are respectively the same as those described for fecal exosomes above. As the solid phase, those generally used in immunological detection methods and methods analogous thereto can be appropriately used, and for example, particles such as magnetic particles and latex particles, plates such as plastic plates, and fibrous substances such as nitrocellulose fibers can be used. For immobilizing the antibody on the solid phase, a conventionally known method or a method analogous thereto can be appropriately employed; the antibody may be directly immobilized on the solid phase by a known method such as a physical adsorption method, a chemical binding method, or a combination thereof, or may be indirectly immobilized by the secondary antibody, protein G, protein A, or biotin-avidin system described above.

Thereby, the presence or absence of a gastrointestinal disease-related protein in the feces or fecal exosomes can be detected as the presence or absence of a signal derived from the labeling substance. In the diagnostic assistance method and diagnostic method of the present invention, it is also preferable to quantify the gastrointestinal disease-related protein from the measured value of the detected signal. Quantification of a gastrointestinal disease-related protein can generally be performed by comparison with a measured value of a standard substance (for example, a standard protein). In this case, for example, the amount of the gastrointestinal disease-related protein in the feces or fecal exosomes can be determined by examining where the obtained measured value is positioned on a standard curve created based on measured values of a standard substance with a known concentration. For example, when MS/MS analysis or LC/MS/MS analysis is used as the detection method according to the present invention, the quantification of the gastrointestinal disease-related protein can be performed, for example, by using the absolute quantified value quantified based on the analysis result, or the relative quantified value (that is, content ratio) obtained from the absolute quantified value and the sample amount provided for the analysis, as a value corresponding to the quantified value.

### [Assistance of Diagnosis, Diagnosis (Determination Step)]

According to the diagnostic assistance method and diagnostic method of the present invention, because the gastrointestinal disease-related protein concentrated in fecal exosomes is detected, the target protein (gastrointestinal disease-related protein) can be detected with high sensitivity at a higher content than the content in total feces, or at a sufficient content for what was below the detection limit in total feces, and can be quantified as necessary. Therefore, the detection result and/or quantification result obtained by the diagnostic assistance method and diagnostic method of the present invention can be used as an indicator for diagnosing the presence or absence of affliction, possibility of affliction, severity, or good or bad follow-up observation of a gastrointestinal disease, depending on the type of gastrointestinal disease (preferably an intestinal disease, more preferably colorectal cancer), based on the presence or absence of detection of the gastrointestinal disease-related protein and/or the amount of the gastrointestinal disease-related protein. Thus, the present invention is a method for such diagnosis or assisting diagnosis, and the method of obtaining data on the presence or absence of detection of a gastrointestinal disease-related protein and/or its amount by the diagnostic assistance method of the present invention can also be expressed as a method for collecting these data for diagnosis by a physician, a method for presenting the data to a physician, or a method for testing for a gastrointestinal disease.

The diagnostic assistance method and diagnostic method of the present invention may, for example, further include a determination step of determining the degree of a gastrointestinal disease in the subject, based on the presence or absence of the gastrointestinal disease-related protein detected in the detection step, and/or a comparison of the amount of the gastrointestinal disease-related protein with a reference amount.

In the determination step, the degree of the gastrointestinal disease is determined, and the determination of "degree" includes the determination of the type and severity of the gastrointestinal disease, the determination of the presence or absence of affliction with the gastrointestinal disease, and the selection of subjects who are predicted to have a high possibility of developing a gastrointestinal disease, and also includes not only distinction from healthy subjects but also distinction from those afflicted with diseases other than gastrointestinal diseases. The "reference amount" to be compared is appropriately set according to the type of gastrointestinal disease and gastrointestinal disease-related protein, the purpose of determination, the subject, and the like, and thus is not particularly limited, but for example, a person skilled in the art can set it as a so-called cut-off value based on the detection method and statistical analysis method described above. The degree of "higher" or "lower" than the reference amount can also be appropriately determined by a person skilled in the art based on a statistical analysis method, according to the type of gastrointestinal disease and gastrointestinal disease-related protein, the purpose of determination, the subject, and the like.

### <Screening method>

The present invention also provides a method for screening a diagnostic marker for a gastrointestinal disease (in this specification, sometimes referred to as "the screening method of the present invention"), comprising:
a step of separating fecal exosomes from feces,
a step of detecting a protein from the separated fecal exosomes, and
a step of selecting a candidate for a diagnostic marker for a gastrointestinal disease from the detected protein, using as an indicator that the protein satisfies any one of the following conditions (a) and (b):
   (a) a content ratio relative to a total amount of the fecal exosomes is 1.5 times or more a content ratio relative to a total amount of the feces,
   (b) the protein is detected only from the separated fecal exosomes and is substantially not detected from the feces.

The present inventors found that, as described above, since gastrointestinal disease-related proteins are frequently and significantly concentrated in fecal exosomes and can be detected with high sensitivity, conversely, proteins concentrated within fecal exosomes can be efficiently screened as candidates for the gastrointestinal disease-related proteins, that is, diagnostic markers useful for the diagnosis of gastrointestinal diseases.

### [Separation Step]

The screening method of the present invention includes a step of separating fecal exosomes from feces (in the screening method of the present invention, "separation step"). As the feces, for example, by respectively adopting feces of a healthy subject and feces of a patient known to be afflicted with a gastrointestinal disease, for example, feces of a healthy subject known not to be afflicted with colorectal cancer and feces of a colorectal cancer patient known to be afflicted with colorectal cancer, and comparing both, it becomes possible to screen for a gastrointestinal disease-related protein specific to the gastrointestinal disease. The method for separating fecal exosomes from the feces, including its preferred embodiments, is the same as the method described for the separation step of the diagnostic assistance method and diagnostic method of the present invention.

### [Detection Step]

The screening method of the present invention includes a step of detecting a protein from the fecal exosomes separated in the separation step (in the screening method of the present invention, "detection step"). The method for detecting the protein, including its preferred embodiments, is the same as the method described for the detection step of the diagnostic assistance method and diagnostic method of the present invention.

However, in the screening method of the present invention, the protein to be detected is not limited to the gastrointestinal disease-related protein, and all proteins may be targeted. As a method for detecting proteins that targets all proteins in this way, for example, a method of comprehensively detecting proteins by performing proteome analysis by MS/MS analysis or LC/MS/MS analysis can be mentioned. For the proteome analysis, for example, analysis software such as Scaffold DIA (manufactured by Proteome Software), DIA-NN (Demichev et al., DIA-NN: neural networks and interference correction enable deep proteome coverage in high throughput, Nat. Methods, 2020, DOI: 10.1038/s41592-019-0638-x) can be used.

In the screening method of the present invention, it is also preferable to quantify the amount of each protein in the feces or fecal exosomes. Such a quantification method, including its preferred embodiments, is the same as the method described in the detection step of the diagnostic assistance method and diagnostic method of the present invention. For example, a relative quantified value calculated by proteome analysis using Scaffold DIA or DIA-NN can be used as the content ratio relative to the sample amount provided for each analysis (that is, the total amount of total feces or the total amount of fecal exosomes), and this can be used in the selection step described below.

### [Selection Step]

The screening method of the present invention includes a step of selecting a candidate for a diagnostic marker for a gastrointestinal disease from the protein detected in the detection step (in the screening method of the present invention, "selection step"), using as an indicator that (a) the content ratio of the protein relative to the total amount of the fecal exosomes is 1.5 times or more (preferably 2 times or more) the content ratio relative to the total amount of the feces, or (b) the protein is detected only from the separated fecal exosomes and is substantially not detected from the feces.

The method for confirming the content ratio of the protein relative to the total amount of fecal exosomes or the total amount of feces, and the presence or absence of detection of the protein from the fecal exosomes or feces, are respectively the same as the respective content ratios and methods for confirming the presence or absence of detection described in the detection step of the diagnostic assistance method and diagnostic method of the present invention, including their preferred embodiments, except that the gastrointestinal disease-related protein is replaced with a protein not limited thereto.

In the screening method of the present invention, for the detection step, for example, it is more preferable to select a candidate for a diagnostic marker for a gastrointestinal disease using as an indicator that it satisfies any one of the following conditions (c) and (d), preferably condition (c), from the detected protein, after performing the separation step and the detection step by adopting the feces of a healthy subject and the feces of a colorectal cancer patient as described above for the feces:
(c) satisfies any one of the conditions (a) and (b) in a colorectal cancer patient,
(d) satisfies any one of the conditions (a) and (b) in a healthy subject.

As for the detection step, among those that satisfy at least one (preferably either, more preferably (c)) of the conditions (c) and (d) above, it is further preferable to select a candidate for a diagnostic marker for a gastrointestinal disease using as an indicator that it satisfies any one of the following conditions (e) and (f):
(e) a Ratio (%) calculated by the following formula: Ratio = (content ratio relative to the total amount of fecal exosomes of a colorectal cancer patient) / (content ratio relative to the total amount of fecal exosomes of a healthy subject) × 100, is 150% or more,
(f) in proteome analysis by DIA-NN, the protein is substantially not detected from the fecal exosomes of a healthy subject, and the content ratio relative to the total amount of fecal exosomes of a colorectal cancer patient is 9.0 × 10³ or more.

Here, in condition (e), the Ratio may be 150% or more, but is more preferably 200% or more, and still more preferably 300% or more. The upper limit of the Ratio is not particularly limited, but is preferably 11000% or less, and more preferably 7000% or less. For example, the Ratio may be 150 to 7000%, or 200 to 7000%.

In condition (f), the content ratio relative to the total amount of fecal exosomes of the colorectal cancer patient may be 9.0 × 10³ or more, but is more preferably 5.0 × 10⁴ or more, and still more preferably 1.0 × 10⁵ or more. Its upper limit is not particularly limited, but is preferably 5.0 × 10⁶ or less, and more preferably 3.0 × 10⁶ or less. For example, the content ratio may be 9.0 × 10³ to 5.0 × 10⁶, 5.0 × 10⁴ to 5.0 × 10⁶, or 1.0 × 10⁵ to 3.0 × 10⁶. Furthermore, the number of the healthy subjects and colorectal cancer patients (that is, the number of specimens) is preferably 3 or more (for example, 3 to 4), and each of the above content ratios is preferably the average value thereof.

The protein selected in the selection step is a gastrointestinal disease-related protein at a high frequency, and therefore can be a candidate for a diagnostic marker for a gastrointestinal disease. The screening method of the present invention preferably also includes a step of further detecting the gastrointestinal disease-related protein from the selected protein (diagnostic marker candidate). As the method for detecting a gastrointestinal disease-related protein from the protein, a known method or a method analogous thereto can be appropriately employed; examples thereof include the immunological detection method using an antibody against the gastrointestinal disease-related protein described above; a method combining MS/MS analysis or LC/MS/MS analysis and proteome analysis; and Western blotting, and two or more of these may be combined.

The candidates for diagnostic markers for a gastrointestinal disease selected in the selection step can be evaluated for their usability as diagnostic markers for the gastrointestinal disease, for example, by analysis of an increase or decrease in expression level related to the gastrointestinal disease, analysis of whether it is a protein derived from a gastrointestinal cancer, or the like, by a conventionally known method or a method analogous thereto, or by comparison between a healthy subject and a gastrointestinal disease patient. Examples of the protein (gastrointestinal disease-related protein) evaluated in this way include the gastrointestinal disease-related proteins mentioned as preferred detection targets in the detection step of the diagnostic assistance method and diagnostic method of the present invention, but are not limited to these.

### <Immunological detection method>

The present invention also provides an immunological detection method for a gastrointestinal disease-related protein (in this specification, sometimes referred to as "the detection method of the present invention"), comprising:
a step of separating fecal exosomes from feces, and
a step of immunologically detecting a gastrointestinal disease-related protein from the separated fecal exosomes.

As described above, because gastrointestinal disease-related proteins are significantly concentrated in fecal exosomes, even for a gastrointestinal disease-related protein that is difficult to detect immunologically in total feces due to low concentration or many contaminants, by performing the step of separating fecal exosomes, it becomes possible to immunologically detect the gastrointestinal disease-related protein with high sensitivity.

### [Separation Step]

The detection method of the present invention includes a step of separating fecal exosomes from feces (in the detection method of the present invention, "separation step"). The feces are as described above and are not particularly limited as long as they can contain the gastrointestinal disease-related protein which is the substance to be detected. The method for separating fecal exosomes from the feces, including its preferred embodiments, is the same as the method described for the separation step of the diagnostic assistance method and diagnostic method of the present invention.

### [Detection Step]

The detection method of the present invention includes a step of immunologically detecting a gastrointestinal disease-related protein from the fecal exosomes separated in the separation step (in the detection method of the present invention, "detection step"). In this detection step, the method for detecting the gastrointestinal disease-related protein is an immunological detection method among the methods described as detection methods in the diagnostic assistance method and diagnostic method of the present invention. The immunological detection method, including its preferred embodiments, is the same as the method described in the diagnostic assistance method and diagnostic method of the present invention.

### <Detection reagent and kit for gastrointestinal disease-related protein>

The present invention provides a detection reagent for a gastrointestinal disease-related protein (in this specification, sometimes simply referred to as "the reagent of the present invention") as a reagent for use in the diagnostic assistance method and diagnostic method of the present invention, the screening method of the present invention, and/or the detection method of the present invention described above. The present invention also provides a kit containing the reagent of the present invention (in this specification, sometimes simply referred to as "the kit of the present invention") as a kit for use in the diagnostic assistance method and diagnostic method of the present invention, the screening method of the present invention, and/or the detection method of the present invention described above.

Examples of the reagent of the present invention include reagents for use therein, depending on the detection method for the gastrointestinal disease-related protein described above, and are not particularly limited, but when the detection method is an immunological detection method, examples include an antibody against the gastrointestinal disease-related protein; the labeling substance or an antibody bound to the labeling substance; a secondary antibody, protein G, or protein A bound to the labeling substance; and the solid phase or an antibody bound to the solid phase, and may be composed of one or two or more of these, or may be a composition containing one or two or more of these. The composition may further contain other components, and examples of the other components are not particularly limited, but include sterile water, physiological saline, surfactants, preservatives, and the like, and one of these or a combination of two or more may be used.

In addition to the reagent of the present invention, the kit of the present invention may further include, for example, as necessary, a suspension solution for feces, a reagent for separating fecal exosomes (for example, a separation medium for density gradient centrifugation), a reagent for detecting fecal exosomes (for example, an antibody against an exosome marker protein or a labeled body in which the antibody is bound to a labeling substance), a reagent for use in various immunological detection methods (for example, when the labeling substance is an enzyme, a substrate solution, a reaction solution, a reaction stop solution), a control (reference), or a combination thereof. The kit of the present invention may also further include instructions for use of the kit.

### [Examples]

Hereinafter, the present invention will be described more specifically based on test examples, but the present invention is not limited to the following test examples. In these test examples, "%" indicating concentration means "weight/volume %, w/v %" unless otherwise specified.

### <Test Example 1> Purification of fecal exosomes from healthy human feces

### (1) Fractionation by density gradient centrifugation

2 to 7 g of feces (pooled sample: a mixture of feces from 20 healthy individuals) was weighed into a tube, 25 mL of phosphate buffer containing a protease inhibitor (manufactured by Roche) was added and suspended, and then the mixture was left to stand on ice for 30 minutes. After suspending again, the mixture was centrifuged at 3,000 × g for 10 minutes, and supernatant 1 was collected. 25 mL of phosphate buffer containing a protease inhibitor (manufactured by Roche) was added again to the precipitate and suspended, then centrifuged at 3,000 × g for 10 minutes, and supernatant 2 was collected and mixed with the previously collected supernatant 1. Thereafter, the mixed supernatant was centrifuged at 3,000 × g for 30 minutes, the obtained supernatant was filtered with filter paper (manufactured by Advantec), and the filtrate was centrifuged at 40,000 × g for 90 minutes. After centrifugation, the supernatant was filtered with a 0.45 µm filter (manufactured by Whatman). The filtrate was then centrifuged at 150,000 × g for 150 minutes to obtain a crude product of fecal exosomes as a precipitate.

Next, density gradient centrifugation was performed on the crude product of fecal exosomes obtained above. First, OptiPrep (60% Iodixanol solution, manufactured by Abbott Diagnostics Technologies) and 8 to 26% sucrose solutions were separately mixed to prepare Iodixanol solutions at concentrations 5 to 40%, and in a centrifuge tube with a diameter of 1.4 cm, 3 mL of 40% Iodixanol solution, 3 mL of 20% Iodixanol solution, 3 mL of 10% Iodixanol solution, and 2.5 mL of 5% Iodixanol solution were layered in this order from the bottom layer to form four layers. On the top layer, a suspension of the crude product (precipitate) of fecal exosomes obtained above suspended in 500 µL of phosphate buffer was placed to make a total of five layers, and centrifuged (density gradient centrifugation) at 100,000 × g for 18 hours. After centrifugation, 1 mL each was collected at every 0.742 cm depth from the top surface to obtain purified fecal exosome fraction solutions of fractions 1 to 12 (Fraction_1 to 12).

Each purified fecal exosome fraction solution was used as is for the detection of fecal exosomes by BLEIA described below. Furthermore, 12 mL of phosphate buffer was added to each purified fecal exosome fraction solution (1 mL) for dilution, then centrifuged at 150,000 × g for 120 minutes, the supernatant was removed, and the precipitate was suspended in 100 µL of phosphate buffer to obtain a purified fecal exosome solution for each fraction.

### (2) Detection of fecal exosomes by BLEIA

For each purified fecal exosome fraction solution, detection of fecal exosomes was performed by BLEIA based on the principle of bioluminescence using a fully automated analyzer BLEIA^{™}-1200 (manufactured by Eiken Chemical Co., Ltd.). Specifically, using each purified fecal exosome fraction solution obtained in (1) above as a sample, as a first reaction, 50 µL of the sample, magnetic particles on which anti-human CD9 antibody (manufactured by Cosmo Bio Co., Ltd., SHI-EXO-M01) was immobilized, and biotinylated anti-human CD63 antibody (manufactured by Cosmo Bio Co., Ltd., SHI-EXO-M02-B) were mixed and reacted at 37°C for 15 minutes. Thereafter, contaminants not captured by the magnetic particles were removed by washing, and 80 µL of Tris buffer containing streptavidin and biotinylated luciferase was added, and a second reaction was performed at 37°C for another 15 minutes. After the second reaction, washing was performed again, and 100 µL of a luminescent substrate solution (manufactured by Eiken Chemical Co., Ltd.) was added to detect the luminescent signal.

The total protein amount of each fraction was quantified by BCA protein assay. Specifically, BCA Working reagent (manufactured by Thermo Fisher Scientific) was added to each purified fecal exosome fraction solution obtained in (1) above, incubated at 37°C for 30 minutes, then cooled to room temperature, and the color development (purple) was measured by absorbance at 562 nm. Using a calibration curve created from the absorbance measured in the same way for a standard protein of known concentration, the total protein amount (ng/µL) in each purified fecal exosome fraction was calculated.

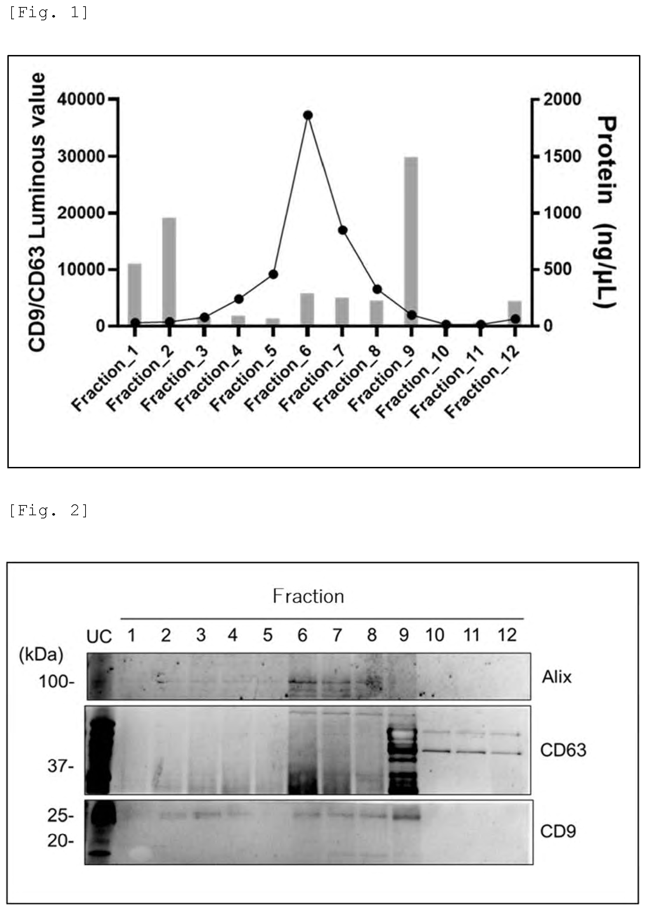

The relationship between the luminescence intensity by BLEIA (CD9/CD63 luminescence intensity, left axis, line graph) and each fraction (Fraction_1 to 12), and the relationship between the total protein amount (Protein (ng/µL), right axis, bar graph) and each fraction (Fraction_1 to 12) are shown together in Fig. 1. As shown in Fig. 1, among the obtained purified fecal exosome fraction solutions, particularly in Fraction_6 to 7, the luminescence intensity corresponding to the fecal exosome surface marker proteins CD9 and CD63 was strong relative to the total protein amount. Therefore, it was confirmed that in the purified fecal exosome fraction solutions obtained in (1) above, fecal exosomes are contained in large amounts in Fraction_6 to 7, and the purity of fecal exosomes is also high in these fractions. Fraction_6 to 7 are the fractions with an Iodixanol concentration of 30 to 45% in the density gradient centrifugation described above.

### (3) Detection of fecal exosomes by Western blotting

For the purified fecal exosome solution of each fraction, detection of fecal exosomes was performed by Western blotting. Specifically, first, 17.5 µL of each purified fecal exosome solution obtained in (1) above was mixed with SDS and DTT, then heated at 95°C for 10 minutes to prepare a Western blot sample. 20 µL of the Western blot sample was subjected to electrophoresis on a 10 to 20% concentration acrylamide gel, then transferred to a PVDF membrane, and reacted with anti-Alix antibody (rabbit-derived, manufactured by Proteintech), anti-CD63 antibody (rabbit-derived, manufactured by Proteintech), or anti-CD9 antibody (rabbit-derived, manufactured by Proteintech) overnight in the cold, each in a separate reaction. Next, the membrane was reacted with HRP-labeled anti-rabbit antibody for 1 hour, then developed with ECL prime (manufactured by Cytiva), and specific bands corresponding to each antibody were detected using the iBright Imaging Systems (manufactured by Thermo Fisher Scientific).

A diagram showing the results of Western blotting is shown in Fig. 2. As shown in Fig. 2, among the obtained purified fecal exosome solutions, in Fraction_6 to 7, bands for Alix, CD63, and CD9, which are surface or internal marker proteins of fecal exosomes, were all confirmed. Therefore, also by the analysis of the purified fecal exosome solutions by Western blotting, it was confirmed that fecal exosomes are contained with high purity in Fraction_6 to 7 obtained in (1) above.

### (4) Detection of fecal exosomes by NanoSight

For the purified fecal exosome solution of each fraction, particle analysis was performed using NanoSight (manufactured by Malvern Panalytical). Specifically, each purified fecal exosome solution obtained in (1) above was diluted 350 to 1000 times with phosphate buffer, and this was sent to NanoSight (manufactured by Malvern Panalytical) with a syringe pump, and the number of particles (particles) up to a particle size of 1000 nm was measured, and its concentration (particles/mL) was calculated. The measurement was performed with n=3, and the average value was adopted.

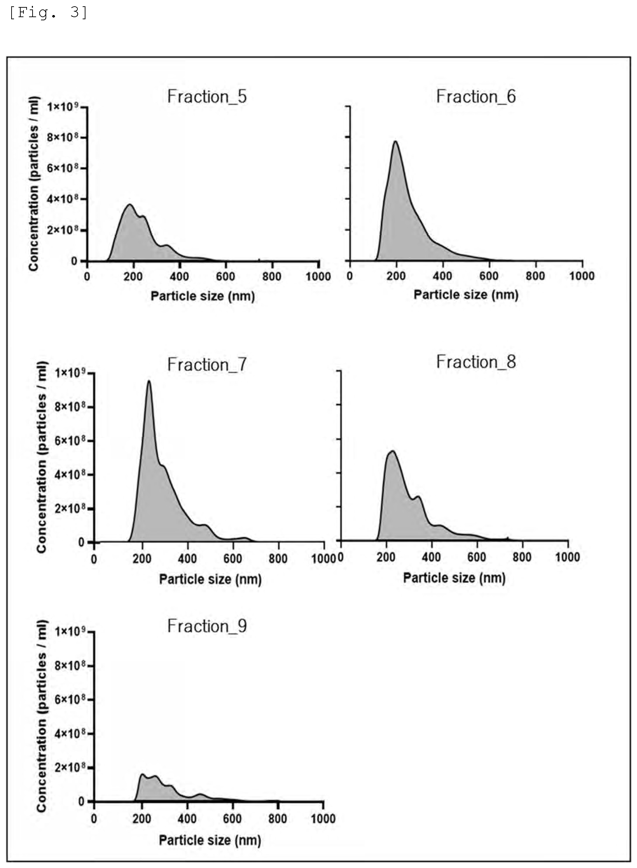

A graph showing the particle size distribution in Fraction_5 to 9 is shown in Fig. 3. As shown in Fig. 3, among the purified fecal exosome solutions obtained in (1), in Fraction_6 to 7, it was confirmed that the particle concentration of particles with a particle size of 100 to 300 nm corresponding to fecal exosomes was high. Therefore, also by the analysis of the purified fecal exosome solutions by NanoSight, it was confirmed that fecal exosomes are contained with high purity in Fraction_6 to 7 obtained in (1) above.

### <Test Example 2> Comparison between fecal exosomes and total feces in healthy individuals

### (1) Proteome analysis by mass spectrometry

Proteome analysis by mass spectrometry was performed using the purified fecal exosome solution of Fraction_6 to 7 obtained in Test Example 1 above as a healthy human fecal exosome sample, and the feces (pooled sample) used in Test Example 1 above as a healthy human total feces sample. Each sample was processed, measured, and analyzed at a contract research organization (Promega Corporation, DIA Proteome Analysis Service) according to the following procedure.

200 ng of each sample was reduced and alkylated, then digested overnight at 37°C by adding 500 ng of Trypsin/Lys-C Mix (manufactured by Promega Corporation). After digestion, the sample was desalted using a reverse-phase spin column (GL Tip SDD, manufactured by GL Sciences Inc.) to prepare an analysis sample. 200 ng of the analysis sample was separated by an UltiMate 3000 RSLCnano LC System (manufactured by Thermo Fisher Scientific), and then subjected to MS/MS analysis (Overlapping window DIA) with a connected Q Exactive HF X (manufactured by Thermo Fisher Scientific). The obtained data was analyzed with Scaffold DIA (manufactured by Proteome Software) to perform identification of proteins (including peptides, the same applies hereinafter) and calculation of quantified values. The identification and calculation of quantified values were performed only for proteins with both Peptide FDR (False Discovery Rate, the same applies hereinafter) and Protein FDR of 1% or less. The quantified values were calculated as relative quantified values (content ratios) relative to the sample amount provided for the analysis by Scaffold DIA software (manufactured by Proteome Software) (no units).

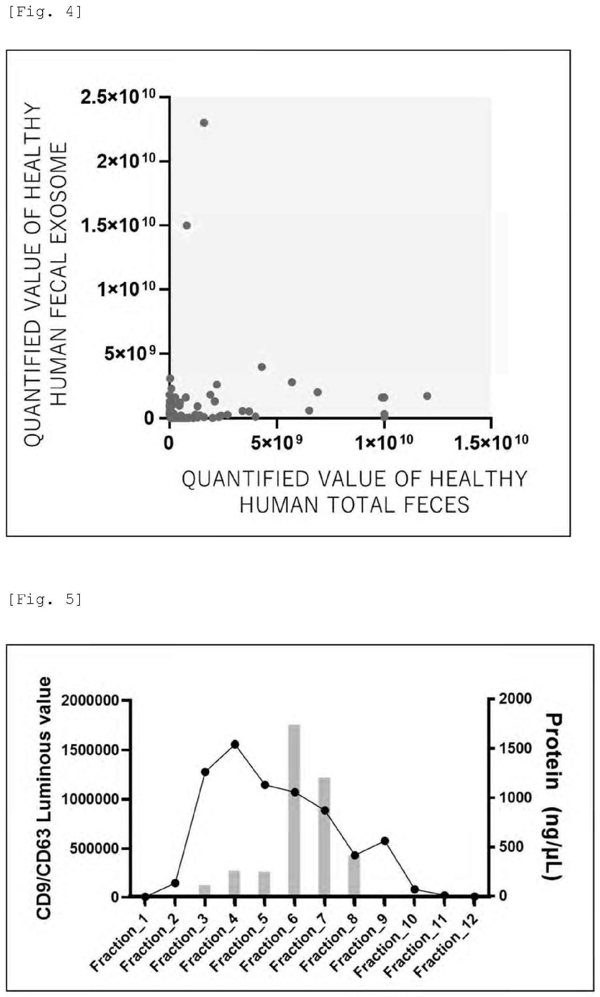

The numbers of proteins identified in healthy human fecal exosomes and healthy human total feces are shown in Table 1 below. As shown in Table 1, a larger variety of proteins were identified in fecal exosomes. Here, 271 types of proteins were common to healthy human fecal exosomes and healthy human total feces, and among these, 89 types had a high content ratio (quantified value) in healthy human fecal exosomes, being two times or more the content ratio in healthy human total feces. Since the same amount of sample was provided for all MS/MS analyses, each quantified value can be read as the content ratio in each sample, and since the detection limit value (3 times the standard deviation) is common, proteins with concentrations lower than the detection limit value are not detected even if present. A plot diagram showing the correlation between the quantified value of proteins in healthy human fecal exosomes and the quantified value of proteins in healthy human total feces for the proteins common to healthy human fecal exosomes and healthy human total feces (271 types) is shown in Fig. 4, but no correlation was confirmed between the quantified value of proteins in healthy human fecal exosomes and the quantified value of proteins in healthy human total feces.

**[Table 1]**

| (Pooled Sample) | Healthy Human Fecal Exosomes | Healthy Human Total Feces |
|---|---|---|
| Protein Count | 558 | 382 |

### (2) Enrichment analysis

For the proteins (89 types) identified in (1) above that were common to healthy human fecal exosomes and healthy human total feces and whose content ratio (quantified value) in healthy human fecal exosomes was two times or more (200% or more) the content ratio in healthy human total feces, or for the proteins (287 types) that were not detected in healthy human total feces but were detected only in healthy human fecal exosomes, an enrichment analysis was performed using The Database for Annotation, Visualization and Integrated Discovery (DAVID) (https://david.ncifcrf.gov/, DAVID 2021 (Dec. 2021), DAVID Knowledgebase v2022q4) to investigate whether proteins derived from the gastrointestinal tract (gastrointestinal tract or its cancer) were significantly abundant (enriched). Tissues or organs with a p-value of less than 0.05 and an FDR of less than 5% were extracted. Among these, the analysis results for tissues or organs belonging to the gastrointestinal tract, together with the corresponding proteins (that is, gastrointestinal tract-derived proteins), are shown in Table 2 below. As shown in Table 2, in the proteins whose content ratio in healthy human fecal exosomes was two times or more the content ratio in healthy human total feces, or in the proteins that were not detected in healthy human total feces but were detected only in healthy human fecal exosomes, proteins derived from the gastrointestinal tract such as Colon, Colon adenocarcinoma, and Small intestine were significantly abundant, and it was confirmed that these proteins are significantly concentrated (enriched) in fecal exosomes. As for other tissues or organs from which the other proteins undergoing the above concentration are derived, for example, the liver was also mentioned as a tissue or organ that satisfies the above conditions (p-value and FDR). However, at the same time, when a similar analysis was performed on proteins that were not detected in healthy human fecal exosomes but were detected only in healthy human total feces, the Count in the liver was high at 76 and satisfied the above conditions (p-value and FDR), confirming that liver-derived proteins cannot be said to be significantly concentrated only in fecal exosomes in general. On the other hand, in the analysis of proteins that were not detected in healthy human fecal exosomes but were detected only in healthy human total feces, no tissue or organ belonging to the gastrointestinal tract was confirmed as satisfying the above conditions (p-value and FDR), confirming that proteins derived from the gastrointestinal tract are significantly concentrated only in fecal exosomes.

**[Table 2]**

| (Pooled Sample) | | | | | |
|---|---|---|---|---|---|
| Gastrointestinal tract | Count | % | PValue | Protein | FDR |
| Content ratio in fecal exosomes is 2-fold or higher | | | | | |
| Colon | 19 | 2.13E+01 | 7.63E-06 | SLC44A4, ANXA2, PTPRJ, CEL, EEF1A1, GPRC5A, CEACAM7, TSPAN8, MEP1A, XPNPEP2, CEACAM5, FTH1, DPEP1, MUC13, PRSS8, ZG16, KIF20B, PPIA, SLC26A3 | 1.51E-04 |
| Colon adenocarcinoma | 5 | 5.62E+00 | 5.02E-04 | CEACAM1, ANXA2, FTH1, PGK1, ZG16 | 6.52E-03 |

| Detected only in fecal exosomes | | | | | |
|---|---|---|---|---|---|
| Colon | 42 | 1.46E+01 | 1.84E-06 | RAB1A, CD82, WDR1, PDCD6, SLC23A1, USH1C, NOXO1, AKAP7, CALML4, SRI, LGALS4, RAB25, CD59, EPS8L3, RAC1, LGALS9, PROM1, TSPAN1, VTA1, LSR, PDZK1, SDHA, TMC4, YWHAZ, RHOA, LGALS9B, SYTL2, MYO1D, SLC9A3, TMIGD1, PLSCR1, CLDN3, STK25, GIPC1, CLDN7, GIPC2, TMBIM1, VDAC2, EZR, PFN1, SERINC2, NOX1 | 1.24E-04 |
| Small intestine | 4 | 1.39E+00 | 1.46E-03 | SLC25A3, BAIAP2L2, MYO15B, STXBP2 | 2.82E-02 |

### <Test Example 3> Comparison between healthy human fecal exosomes and colorectal cancer patient fecal exosomes 1

### (1) Purification of exosomes from feces of colorectal cancer patient

Since it was confirmed by Test Example 1 that fecal exosomes can be purified with high purity from feces by the method described in that test example, fractionation of the feces of a colorectal cancer patient by density gradient centrifugation was performed in the same manner as in Test Example 1, except that feces collected from a colorectal cancer patient (1 specimen) were used as the feces, and fecal exosomes were detected by BLEIA, Western blotting, and NanoSight.

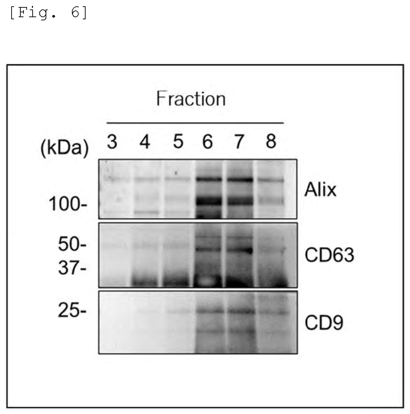

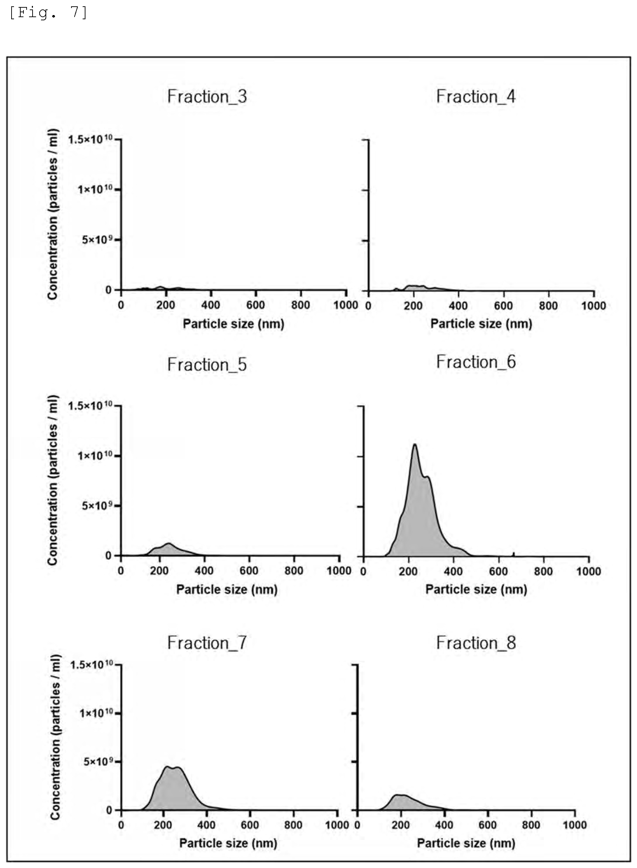

Fig. 5 shows the relationship between the luminescence intensity by BLEIA (CD9/CD63 luminescence intensity, left axis, line graph) and each fraction (Fraction_1 to 12), and the relationship between the total protein amount by BCA protein assay (Protein (ng/µL), right axis, bar graph) and each fraction (Fraction_3 to 8). Fig. 6 shows the results of Western blotting performed on the purified fecal exosome solution of Fraction_3 to 8. Furthermore, a graph showing the particle size distribution in the purified fecal exosome solution of Fraction_3 to 8 is shown in Fig. 7. As shown in Figs. 5 to 7, since it was confirmed that fecal exosomes are contained with high purity in Fraction_6 to 7 of the purified fecal exosome fraction solutions, the purified fecal exosome solution of Fraction_6 to 7 was used as a colorectal cancer patient fecal exosome sample.

### (2) Proteome analysis by mass spectrometry

A healthy human fecal exosome sample (purified fecal exosome solution of Fraction_6 to 7) was obtained in the same manner as in Test Example 1, except that different feces (pooled sample: a mixture of feces from 20 healthy individuals different from those in Test Example 1) were used as the sample. Proteome analysis by mass spectrometry was performed on this and the colorectal cancer patient fecal exosome sample obtained in (1) above. Each sample was processed, measured, and analyzed at a contract research organization (Promega Corporation, DIA Proteome Analysis Service) according to the following procedure.

200 ng of each sample was reduced and alkylated, then digested overnight at 37°C by adding 500 ng of Trypsin/Lys-C Mix (manufactured by Promega Corporation). After digestion, the sample was desalted using a reverse-phase spin column (GL Tip SDD, manufactured by GL Sciences Inc.) to prepare an analysis sample. 200 ng of the analysis sample was separated by an UltiMate 3000 RSLCnano LC System (manufactured by Thermo Fisher Scientific), and then subjected to MS/MS analysis (DIA MS) with a connected Q Exactive HF X (manufactured by Thermo Fisher Scientific). The obtained data was analyzed with DIA-NN (Demichev et al., DIA-NN: neural networks and interference correction enable deep proteome coverage in high throughput, Nat. Methods, 2020, DOI: 10.1038/s41592-019-0638-x) to perform identification of proteins and calculation of quantified values. The identification and calculation of quantified values were performed only for proteins with both Precursor FDR and Protein FDR of 1% or less. The quantified values were calculated as relative quantified values (that is, content ratios in each sample) relative to the sample amount provided for the analysis by DIA-NN (no units) .

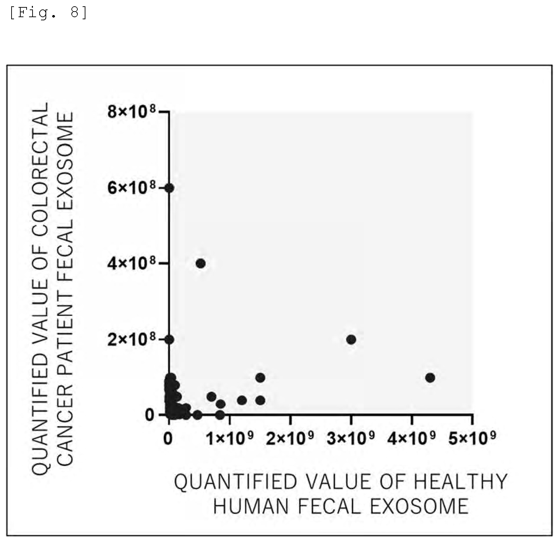

The numbers of proteins identified in healthy human fecal exosomes and colorectal cancer patient fecal exosomes are shown in Table 3 below. The number of proteins identified in colorectal cancer patient fecal exosomes by the above analysis was 1200, which was even greater than the 457 proteins identified in healthy human fecal exosomes by the same analysis. A plot diagram showing the correlation between the quantified value of proteins in colorectal cancer patient fecal exosomes and the quantified value of proteins in healthy human fecal exosomes for the proteins common to healthy human fecal exosomes and colorectal cancer patient fecal exosomes (442 types) is shown in Fig. 8. In Fig. 8, the correlation coefficient (r) between the quantified value of proteins in colorectal cancer patient fecal exosomes and the quantified value of proteins in healthy human fecal exosomes was 0.2736, and no correlation was confirmed between the two.

**[Table 3]**

| | Healthy Human Fecal Exosomes | Colorectal Cancer Patient Fecal Exosomes |
|---|---|---|
| Protein Count | 457 | 1200 |

### (3) Enrichment analysis

For the 758 types of proteins identified in (2) above and detected only in colorectal cancer patient fecal exosomes, an enrichment analysis was performed in the same manner as in (2) of Test Example 2 to investigate whether proteins derived from the gastrointestinal tract (gastrointestinal tract or its cancer) were significantly abundant (enriched). When tissues or organs with a p-value of less than 0.05 and an FDR of less than 5% were extracted, it was confirmed that in colorectal cancer patient fecal exosomes, similarly to the healthy human fecal exosomes above, a significant concentration of proteins derived from the gastrointestinal tract such as Colon, Small intestine, and Colon adenocarcinoma was occurring.

### <Test Example 4> Comparison between fecal exosomes and total feces in healthy individuals and cancer patients

### (1) Purification of exosomes from healthy human feces and colorectal cancer patient feces

Healthy human fecal exosome samples (purified fecal exosome solutions of Fraction_6 to 7) were obtained in the same manner as in Test Example 1, except that 4 specimens of feces collected from 4 healthy individuals (Healthy individuals 1 to 4) were used as healthy human feces. Colorectal cancer patient fecal exosome samples (purified fecal exosome solutions of Fraction_6 to 7) were obtained in the same manner as in (1) of Test Example 3, except that 4 specimens of feces collected from 4 colorectal cancer patients (Colorectal cancer 1 to 4) were used as the feces.

### (2) Proteome analysis by mass spectrometry

Proteome analysis by mass spectrometry was performed in the same manner as in (2) of Test Example 3, using each of the feces used in (1) above as a healthy human total feces sample and a colorectal cancer patient total feces sample, respectively, and these along with each healthy human fecal exosome sample and each colorectal cancer patient fecal exosome sample obtained in (1) above.

The numbers of proteins identified in each healthy human fecal exosome and healthy human total feces are shown in Table 4 below, and the numbers of proteins identified in each colorectal cancer patient fecal exosome and colorectal cancer patient total feces are shown in Table 5 below. For the specimen of Colorectal cancer 4, identification of the number of proteins in colorectal cancer patient total feces was not performed. In all specimens, similarly to (1) of Test Example 2 and (2) of Test Example 3, it was confirmed that the number of proteins identified in each fecal exosome was greater than the number of proteins identified in total feces by the same analysis.

**[Table 4]**

| (Healthy Human Specimen) | | Healthy Individual 1 | Healthy Individual 2 | Healthy Individual 3 | Healthy Individual 4 |
|---|---|---|---|---|---|
| Protein Count | Healthy Human Fecal Exosomes | 326 | 719 | 634 | 635 |
| | Healthy Human Total Feces | 242 | 432 | 378 | 347 |

**[Table 5]**

| (Cancer Patient Specimen) | | Colorectal Cancer 1 | Colorectal Cancer 2 | Colorectal Cancer 3 | Colorectal Cancer 4 |
|---|---|---|---|---|---|
| Protein Count | Colorectal Cancer Patient Fecal Exosomes | 1631 | 729 | 1039 | 981 |
| | Colorectal Cancer Patient Total Feces | 1182 | 551 | 838 | - |

### (3) Enrichment analysis

In a total of 7 specimens from Healthy individuals 1 to 4 and Colorectal cancer 1 to 3, for the proteins identified in (2) above that were common to each fecal exosome and each total feces and whose content ratio (quantified value) in fecal exosomes was two times or more (200% or more) the content ratio in total feces, or, in a total of 7 specimens from Healthy individuals 1 to 4 and Colorectal cancer 1 to 3, for the proteins identified in (2) above that were not detected in total feces but were detected only in fecal exosomes, an enrichment analysis was performed in the same manner as in (2) of Test Example 2 to investigate whether proteins derived from the gastrointestinal tract (gastrointestinal tract or its cancer) were significantly abundant (enriched). Tissues or organs with a p-value of less than 0.05 and an FDR of less than 5% were extracted, and among these, the analysis results for tissues or organs belonging to the gastrointestinal tract, together with the corresponding proteins (that is, gastrointestinal tract-derived proteins), are shown in Tables 6 to 9 below. As shown in Tables 6 to 9, in proteins whose content ratio in fecal exosomes was two times or more the content ratio in total feces (Tables 6, 8), or in proteins that were not detected in total feces but were detected only in fecal exosomes (Tables 7, 9), in both healthy human and colorectal cancer patient specimens, similarly to (2) of Test Example 2 and (3) of Test Example 3, proteins derived from the gastrointestinal tract were significantly abundant, and it was confirmed that these proteins are significantly concentrated (enriched) in fecal exosomes. In this analysis, similarly to (2) of Test Example 2, the liver, etc., were also mentioned as other tissues or organs that satisfy the above conditions (p-value and FDR), but when a similar analysis was performed on proteins that were not detected in fecal exosomes but were detected only in total feces, no tissue or organ belonging to the gastrointestinal tract that satisfied the above conditions (p-value and FDR) was confirmed, whereas in the liver, the Count was high at 90 or more and satisfied the above conditions (p-value and FDR), confirming that liver-derived proteins cannot be said to be significantly concentrated only in fecal exosomes in general. A similar analysis was performed for proteins derived from tissues or organs other than the gastrointestinal tract and liver, but none of them satisfied the above conditions (p-value and FDR), and the only tissue or organ for which a significant concentration of proteins was confirmed only in fecal exosomes common to all specimens in this analysis was the gastrointestinal tract.

**[Table 6]**

| Sample | Gastrointestinal tract | Count | % | PValue | Protein | FDR |
|---|---|---|---|---|---|---|
| Content ratio in fecal exosomes is 2-fold or higher | | | | | | |
| Healthy individual 1 | Colon adenocarcinoma | 4 | 9.52E+00 | 6.58E-04 | CEACAM1, ANXA2, FTH1, ZG16 | 3.21E-02 |
| | Colon | 10 | 2.38E+01 | 8.23E-04 | LGALS4, ANXA2, CEACAM7, XPNPEP2, FTH1, DPEP1, ZG16, SLC26A3, PPIA, ENPP7 | 3.21E-02 |
| Healthy individual 2 | Colon | 24 | 2.20E+01 | 3.43E-07 | SLC44A4, HSP90AB1, ANXA2, TPM1, PTPRJ, CEL, LGALS4, FABP1, GPRC5A, ADGRG7, CEACAM7, TSPAN8, FAM151A, XPNPEP2, FTH1, DPEP1, HLA-DRA, VDAC2, MUC13, EPS8L3, ZG16, PPIA, SLC26A3, ENPP7 | 8.75E-06 |
| | Colon carcinoma | 13 | 1.19E+01 | 9.06E-05 | HSPA8, SLC25A3, HSP90AB1, HSPA5, GSTP1, ENO1, RAB10, LGALS4, PKM, GPRC5A, TSPAN8, SLC25A5, HSPA1A | 1.15E-03 |
| | Colon adenocarcinoma | 5 | 4.59E+00 | 1.20E-03 | SLC25A3, CEACAM1, ANXA2, FTH1, ZG16 | 8.76E-03 |
| | Intestine | 4 | 3.67E+00 | 2.25E-03 | VIL1, SLPI, DEFA5, MUC3A | 1.53E-02 |
| | Small intestine | 9 | 8.26E+00 | 2.99E-03 | VIL1, SLC44A4, UBB, DMBT1, ITLN1, ITLN2, MUC3A, ENPP7, PLS1 | 1.82E-02 |
| Healthy individual 3 | Colon | 22 | 2.27E+01 | 6.60E-07 | SLC44A4, HSP90AB1, ANXA2, CEL, LGALS4, FABP1, GPRC5A, CEACAM7, TSPAN8, XPNPEP2, CEACAM5, FTH1, DPEP1, MUC13, PRSS8, EPS8L3, ZG16, LGALS9, SMPDL3B, PPIA, SLC26A3, ENPP7 | 1.08E-05 |
| | Colon carcinoma | 12 | 1.24E+01 | 1.31E-04 | LGALS4, RAB10, HSPA8, GPRC5A, HSP90AB1, TSPAN8, GSTP1, CALM1, ALDOA, SLC25A5 | 1.17E-03 |
| | Colon adenocarcinoma | 4 | 4.12E+00 | 8.12E-03 | CEACAM1, ANXA2, FTH1, ZG16 | 4.42E-02 |
| Healthy individual 4 | Colon | 21 | 2.76E+01 | 4.23E-08 | SLC44A4, ANXA2, CD82, WDR1, CEL, OLFM4, LGALS4, PLSCR1, CLDN3, GPRC5A, CEACAM7, TSPAN8, GIPC1, MUC13, PRSS8, EPS8L3, ZG16, PFN1, EZR, PPIA, SLC26A3 | 3.85E-06 |

**[Table 7]**

| Sample | Gastrointestinal tract | Count | % | PValue | Protein | FDR |
|---|---|---|---|---|---|---|
| Detected only in fecal exosomes | | | | | | |
| Healthy individual 1 | Colon | 34 | 1.67E+01 | 9.46E-08 | AHCYL1, SLC44A4, CD82, PDCD6, SLC23A1, PTPRJ, SRI, FUT3, RAB25, TSPAN8, MUC13, MS4A12, PRSS8, EPS8L3, STX3, RAC1, LGALS9, RNASE3, CEL, LYZ, TMC4, MYO1D, SLC9A3, TMIGD1, PLSCR1, CLDN3, GPRC5A, DDAH1, ARPC2, GIPC1, VDAC2, EZR, PFN1, NOX1 | 6.76E-06 |
| Healthy individual 2 | Colon | 56 | 1.33E+01 | 4.76E-07 | AHCYL1, CD82, WDR1, PDCD6, SLC23A1, CALML4, SERPINA4, RAB22A, CNDP2, ARHGDIA, CHP2, MS4A12, RAC1, LGALS9, RPS10, AKR1A1, LSR, RNASE3, PDZK1, SDHA, TMC4, RHOA, LGALS9B, SYTL2, SLC9A3, TMIGD1, PLSCR1, CLDN3, DDAH1, TMBIM1, EZR, PFN1, FBP1, CSNK1G2, FBP2, RAB1A, CD151, AK1, USH1C, NOXO1, AKAP7, SRI, RAB25, CD59, STX3, PROM1, TSPAN1, SLC16A1, VTA1, MYO1D, STK25, GIPC1, GIPC2, SLC28A2, NOX1, LNX2 | 2.24E-05 |
| Healthy individual 3 | Colon | 49 | 1.27E+01 | 9.36E-06 | AHCYL1, CD82, WDR1, PDCD6, SLC23A1, CALML4, SERPINA4, RAB22A, ARHGDIA, RPS6KA1, MS4A12, RAC1, CD177, LSR, RNASE3, PDZK1, TMC4, RHOA, LGALS9B, SYTL2, SLC9A3, TMIGD1, PLSCR1, CLDN3, DDAH1, TMBIM1, VDAC2, EZR, PFN1, CSNK1G2, RAB1A, CD151, USH1C, NOXO1, AKAP7, SRI, FUT3, SULT1A1, RAB25, CD59, STX3, PROM1, TSPAN1, MYO1D, FERMT1, STK25, GIPC1, GIPC2, NOX1 | 2.05E-04 |
| | Small intestine | 4 | 1.04E+00 | 3.26E-03 | SLC25A3, BAIAP2L2, MYO15B, STXBP2 | 3.36E-02 |
| Healthy individual 4 | Colon | 51 | 1.21E+01 | 1.10E-05 | AHCYL1, HSP90AB1, GFM2, PDCD6, CLTC, CALML4, RAB22A, CNDP2, MS4A12, RAC1, LGALS9, RPS10, CD177, LSR, PDZK1, TMC4, RHOA, LGALS9B, SYTL2, SLC9A3, SPINT1, TMBIM1, VDAC2, CSNK1G2, PAFAH1B1, OTUB1, RAB1A, USH1C, NOXO1, AKAP7, SRI, FUT3, SULT1A1, ADGRG7, RAB25, MYH14, CD59, STX3, PROM1, TSPAN1, REG4, VTA1, LYZ, MYO1D, FERMT1, ARPC2, FAM151A, STK25, GIPC2, SLC28A2, NOX1 | 1.96E-04 |
| | Intestine | 7 | 1.67E+00 | 4.17E-04 | SLC36A1, SLC15A1, SLPI, MYO1A, ATP8B1, DEFA5, MUC3A | 5.71E-03 |
| | Colon carcinoma | 25 | 5.95E+00 | 1.28E-03 | GUCY2C, SLC25A3, DDX3X, HSP90AB1, YWHAB, GSTP1, RAB22A, CNDP2, RAB21, PPP1CC, LASP1, GPA33, CHP1, MYH14, YWHAG, ANXA3, VTA1, MINK1, VPS37B, OCLN, HNRNPA2B1, KRAS, TAGLN2, SLC25A5, PLEC | 1.42E-02 |

**[Table 8]**

| Sample | Gastrointestinal tract | Count | | PValue | Protein | FDR |
|---|---|---|---|---|---|---|
| Content ratio in fecal exosomes is 2-fold or higher | | | | | | |
| Colorectal cancer 1 | Colon | 71 | 1.51E+01 | 4.30E-11 | RPL4, LGALS3BP, AHCYL1, SLC44A4, HSP90AB1, CD82, WDR1, PDCD6, RPLPO, CLTC, HNRNPU, PTPRJ, CALML4, CNDP2, LGALS4, C8G, NARS1, ARHGDIA, FTH1, MUC13, ACADM, RAC1, LGALS9, RPS10, CD177, AKR1A1, ACSL5, APOA4, RNASE3, OLFM4, SDHA, NIPSNAP2, HADHB, GPRC5A, CEACAM7, MEP1A, CEACAM5, TCP1, DPEP1, VDAC2, ZG16, EZR, SMPDL3B, PFN1, USH1C, TM9SF3, C2, FUT3, SULT1A1, ADGRG7, TSPAN8, AGR2, CLCA1, CD59, EPS8L3, RPL18, TSPAN1, REG4, IDH2, PA2G4, SOD2, PGA4, MYO1D,ARPC2, FAM151A, XPNPEP2, RPL27A, IMPDH2, HLA-DRA, SLC26A3, KLKB1 | 1.04E-09 |
| | Small intestine | 30 | 6.38E+00 | 8.56E-07 | SLC44A4, MTTP, ITLN2, GBA1, HMGB1, CLU, C2, FUT3, ATP5F1A, RPS15A, UBB, DMBT1, CLCA1, APOB, CD300LF, PLTP, PLS1, MUC17, CYBRD1, MUC3A, SERPINB5, NIPSNAP2, NAALADL1, VIL1, FOLH1, MEP1B, MEP1A, LCT, RAN, HLA-DRB1 | 1.38E-05 |
| | Colon adenocarcinoma | 11 | 2.34E+00 | 3.08E-05 | SULT1A1, PYGB, SLC25A3, CEACAM1, RPS15A, ATIC, FTH1, RPS3, ZG16, GAPDH, RAN | 3.76E-04 |
| | Colon carcinoma | 32 | 6.81E+00 | 3.10E-05 | SLC25A3, COPA, DDX3X, HSP90AB1, YWHAB, HNRNPU, CNDP2, LGALS4, TSPAN8, RPS18, EPCAM, GPA33, CHP1, CKB, PSMF1, CCT7, YWHAG, CYB5B, HSPA9, CAP1, PARP4, PDCD6IP, HSPA4, KCNAB2, ILF2, RAB10, GPRC5A, EIF2S3, HNRNPK, SLC25A5, ALDH9A1, HSPA1A | 3.76E-04 |
| | Intestine | 8 | 1.70E+00 | 1.19E-04 | VIL1, MEP1B, MYO1A, ANPEP, SI, DEFA5, APOA4, MUC3A | 1.22E-03 |
| Colorectal cancer 2 | Small intestine | 11 | 7.97E+00 | 9.15E-04 | VIL1, ATP5F1A, SLC44A4, UBB, DMBT1, RBP2, ITLN1, ITLN2, MUC3A, XDH, PLS1 | 7.06E-03 |
| | Colon | 20 | 1.45E+01 | 1.11E-03 | LGALS3BP, SLC44A4, HSP90AB1, ANXA2, GDA, CEL, RNASE3, OLFM4, LGALS4, TMIGD1, CEACAM7, XPNPEP2, CEACAM5, DPEP1, MUC13, ZG16, LGALS9, EZR, SMPDL3B, SLC26A3 | 7.97E-03 |
| | Intestine | 4 | 2.90E+00 | 4.18E-03 | VIL1, SLPI, DEFA5, MUC3A | 2.66E-02 |
| | Colon carcinoma | 11 | 7.97E+00 | 7.09E-03 | LGALS4, HSPA8, SLC25A3, PKM, HSP90AB1, HSPA5, ANXA3, GSTP1, HNRNPA2B1, ALDOA, SLC25A5 | 4.25E-02 |
| | Colon mucosa | 4 | 2.90E+00 | 8.90E-03 | CEACAM7, MUC12, MUC3A, MUC4 | 4.37E-02 |
| Colorectal cancer 3 | Colon | 26 | 1.51E+01 | 7.08E-05 | LGALS3BP, RPL5, HSP90AB1, USH1C, SRI, LGALS4, TSPAN8, FTH1, MUC13, CD59, RPS2, RPS10, ANXA2, RNASE3, CEL, OLFM4, LYZ, SDHA, PGA4, CEACAM7, HLA-DRA, VDAC2, ZG16, EZR, SLC26A3, PPIA | 1.10E-03 |
| | Colon adenocarcinoma | 6 | 3.49E+00 | 7.42E-04 | CEACAM1, ANXA2, FTH1, RPS3, ZG16, DDOST | 1.02E-02 |
| | Colon carcinoma | 14 | 8.14E+00 | 1.46E-03 | RPL5, PRPS1, PARP4, HSP90AB1, YWHAB, ANXA3, RAB10, LGALS4, TSPAN8, EPCAM, GPA33, SAFB2, CKB, SLC25A5 | 1.89E-02 |

**[Table 9]**

| Sample | Gastrointestinal tract | Count | % | PValue | Protein | FDR |
|---|---|---|---|---|---|---|
| Detected only in fecal exosomes | | | | | | |
| Colorectal cancer 1 | Colon | 73 | 1.13E+01 | 6.41E-06 | SLC35B2, RPL5, RPL3, SLC23A1, APIP, RAB22A, PPP2R1A, RUVBL2, RPS6KA1, GNPNAT1, VPS35, UPP1, APPL2, RPS9, ACOT7, RPL21, RPL13A, LSR, ATP11B, PDZK1, TMC4, RHOA, LGALS9B, SYTL2, SLC9A3, TMIGD1, PLSCR1, POR, CLDN3, TMBIM1, TFF3, RPL24, RPL28, EPHA2, PAFAH1B1, OTUB1, RAB1A, CD151, TMED10, HPGD, AK1, NOXO1, VPS26A, HSD17B11, ADH6, RAB25, HSD17B2, TM4SF20, NCKAP1L, STX3, RPS2, PROM1, NDUFV1, NQO1, SLC16A1, PLEKHA1, VTA1, AKR1C3, RPL35A, H1-0, TM9SF2, FERMT1, PFKL, DHRS9, EIF3L, STK25, GIPC1, LPCAT3, GIPC2, NOSTRIN, CORO7, SLC28A2, NOX1 | 1.97E-04 |
| Colorectal cancer 2 | Colon | 47 | 1.28E+01 | 1.49E-05 | AHCYL1, CD151, CD82, WDR1, PDCD6, SLC23A1, USH1C, AKAP7, CALML4, SRI, CNDP2, SULT1A1, RAB25, TSPAN8, ARHGDIA, FTH1, CHP2, CD59, MS4A12, EPS8L3, STX3, RAC1, TSPAN1, AKR1C3, AKR1A1, RPL35A, LSR, PDZK1, TMC4, RHOA, LGALS9B, SYTL2, MYO1D, SLC9A3, PLSCR1, CLDN3, GPRC5A, STK25, GIPC1, CLDN7, GIPC2, TMBIM1, VDAC2, SLC28A2, PFN1, PPIA, NOX1 | 4.45E-04 |
| | Colon adenocarcinoma | 10 | 2.72E+00 | 2.78E-05 | SULT1A1, RPS15A, FTH1, CLDN7, ANXA13, RPS3, PGK1, ANXA7, UBE2V1, RAN | 6.77E-04 |
| Colorectal cancer 3 | Colon | 70 | 1.40E+01 | 2.00E-09 | RPL4, AHCYL1, SLC44A4, RPL3, CD82, WDR1, PDCD6, RPLPO, CLTC, APIP, HNRNPU, CALML4, RAB22A, C8G, NARS1, PPP2R1A, RPS6KA1, CHP2, VPS35, PRSS8, MS4A12, RAC1, LGALS9, CD177, RPS9, RPL21, RPL13A, ACSL5, LSR, PDZK1, TMC4, RHOA, SYTL2, NIPSNAP2, SLC9A3, TMIGD1, PLSCR1, CLDN3, GPRC5A, ACOX1, CLDN7, TMBIM1, TUT1, RAB1A, CD151, TMED10, AK1, ATP10B, VPS26A, HSD17B11, TM9SF3, FUT3, SULT1A1, RAB25, STX3, EPS8L3, TSPAN1, REG4, VTA1, TM9SF2, MYO1D, PTPRB, PFKL, DHRS9, GIPC1, LPCAT3, GIPC2, NOSTRIN, NOX1, LNX2 | 1.20E-07 |
| | Colon adenocarcinoma | 11 | 2.20E+00 | 5.34E-05 | SULT1A1, PYGB, SLC25A3, RPS15A, RPL3, ADGRE5, NOS2, NCF2, CLDN7, ANXA13, ANXA7 | 1.78E-03 |

### <Test Example 5> Comparison between healthy human fecal exosomes and colorectal cancer patient fecal exosomes 2

First, Pathology data from "The Human Protein Atlas database (https://www.proteinatlas.org/)" which is a database of protein staining profiles and their staining levels in human tumor tissues based on immunohistochemistry, was downloaded (June 16, 2023), and colorectal cancer data was obtained from them. From the obtained data, a ratio (n/N) was calculated by dividing the total number of proteins (n) with staining levels of High, Medium, or Low by the total number of stainings (N), and those for which this ratio exceeded 0.5 were selected as colorectal cancer-related proteins. The number of colorectal cancer-related proteins selected from the database was 9723 proteins.

Next, proteins that matched the 9723 proteins above were extracted from the proteins identified in each healthy human fecal exosome and colorectal cancer patient fecal exosome in (2) of Test Example 4; furthermore, from among these, proteome analysis by mass spectrometry was performed in the same manner as in (2) of Test Example 3 to calculate quantified values (content ratios) for proteins that were common to healthy human fecal exosomes and healthy human total feces and for which the content ratio in healthy human fecal exosomes was two times or more (200% or more) the content ratio (quantified value) in healthy human total feces; proteins that were not detected in healthy human total feces but were detected only in healthy human fecal exosomes; proteins that were common to colorectal cancer patient fecal exosomes and colorectal cancer patient total feces and for which the content ratio in colorectal cancer patient fecal exosomes was two times or more (200% or more) the content ratio in colorectal cancer patient total feces; and proteins that were not detected in colorectal cancer patient total feces but were detected only in colorectal cancer patient fecal exosomes, corresponding to at least one of these.

For the gastrointestinal disease-related proteins on which analysis was performed, the ratio (Ratio: quantified value in colorectal cancer patient fecal exosomes / quantified value in healthy human fecal exosomes × 100 (%)) between the average value of quantified values in healthy human fecal exosomes (average value of Healthy individuals 1 to 4; the quantified value of a specimen in which the protein was not identified was calculated as 0) and the average value of quantified values in colorectal cancer patient fecal exosomes (average value of Colorectal cancer 1 to 3; the quantified value of a specimen in which the protein was not identified was calculated as 0) was calculated. A list of proteins for which the ratio (Ratio) was 150% (1.5 times) or more, along with their average quantified values in each fecal exosome and the ratio (Ratio), is shown in Tables 10 to 12. A list of gastrointestinal disease-related proteins on which analysis was performed that were not detected in any of specimens 1 to 4 of healthy human fecal exosomes and were detected only in at least one of the colorectal cancer patient fecal exosomes of Colorectal cancer 1 to 3, and their average quantified values in each fecal exosome (average value of Colorectal cancer 1 to 3; the quantified value of a specimen in which the protein was not identified was calculated as 0), is shown in Tables 13 to 15.

Among the gastrointestinal disease-related proteins described in Tables 10 to 12, proteins commonly detected from fecal exosomes in two or more colorectal cancer patient specimens are shown in Fig. 9, and among the gastrointestinal disease-related proteins described in Tables 13 to 15, proteins commonly detected from fecal exosomes in two or more colorectal cancer patient specimens are shown in Fig. 10. Furthermore, among the gastrointestinal disease-related proteins described in Tables 10 to 15, proteins commonly detected from fecal exosomes in all colorectal cancer patient specimens (4 specimens) are shown in Fig. 11.

In both healthy individuals and colorectal cancer patients, gastrointestinal disease-related proteins are concentrated in fecal exosomes (that is, the quantified value (content ratio) in each fecal exosome is two times or more the quantified value (content ratio) in each total feces, or they are not detected in each total feces but detected only in each fecal exosome); however, as shown in Tables 10 to 12, it was confirmed that in the fecal exosomes of colorectal cancer patients, many gastrointestinal disease-related proteins are concentrated, and the content is significantly higher than in the fecal exosomes of healthy individuals (that is, the Ratio is 150% or more). Furthermore, as shown in Tables 13 to 15, in the fecal exosomes of colorectal cancer patients, more gastrointestinal disease-related proteins were concentrated and detected than in the fecal exosomes of healthy individuals (that is, they were not detected in healthy human fecal exosomes but were concentrated only in colorectal cancer patient fecal exosomes). From these results, it was confirmed that gastrointestinal disease-related proteins (for example, colorectal cancer-related proteins) are concentrated in fecal exosomes (especially in the fecal exosomes of gastrointestinal disease patients (for example, colorectal cancer patients)). Although gastrointestinal disease-related proteins may differ depending on the degree of the disease and individual differences and are not necessarily common among all patients, it was confirmed that at least, as shown in Figs. 9 to 11, many gastrointestinal disease-related proteins are commonly concentrated in the fecal exosomes of colorectal cancer patients among specimens.

**[Table 10]**

| Protein | Quantified value of healthy human fecal exosomes (average) | Quantified value of colorectal cancer patient fecal exosomes (average) | Ratio | Protein | Quantified value of healthy human fecal exosomes (average) | Quantified value of colorectal cancer patient fecal exosomes (average) | Ratio |
|---|---|---|---|---|---|---|---|
| ZNF256 | 2.85E+05 | 1.68E+06 | 592% | S100A11 | 1.76E+05 | 5.72E+05 | 325% |
| ZMPSTE24 | 5.69E+04 | 3.86E+05 | 678% | RPS7 | 8.74E+03 | 5.53E+05 | 6327% |
| YWHAH | 4.30E+05 | 1.27E+06 | 295% | RPS6KA1 | 1.50E+04 | 2.35E+05 | 1567% |
| YWHAG | 6.91E+05 | 1.25E+06 | 181% | RPS3 | 1.37E+05 | 9.94E+05 | 724% |
| WDR1 | 6.37E+05 | 1.84E+06 | 288% | RPS23 | 5.61E+04 | 3.47E+05 | 618% |
| VILL | 1.02E+05 | 3.64E+05 | 357% | RPS16 | 4.17E+04 | 1.68E+06 | 4039% |
| VASP | 6.29E+04 | 1.06E+06 | 1691% | RPS10 | 5.71E+04 | 3.93E+05 | 689% |
| VAMP8 | 2.13E+06 | 4.95E+06 | 233% | RPL35 | 1.05E+05 | 2.81E+05 | 269% |
| USP5 | 2.97E+04 | 7.42E+04 | 250% | RPL30 | 2.09E+05 | 9.95E+05 | 477% |
| UGDH | 1.03E+05 | 6.26E+05 | 608% | RNPEP | 1.35E+06 | 4.51E+06 | 335% |
| UBE2D3 | 1.31E+05 | 2.67E+05 | 203% | RNF128 | 7.49E+04 | 1.20E+05 | 161% |
| TUBB4B | 4.13E+05 | 1.83E+06 | 444% | RNASE3 | 1.69E+06 | 9.26E+06 | 549% |
| TSPAN6 | 4.74E+05 | 1.74E+06 | 368% | RNASE1 | 3.11E+05 | 2.42E+06 | 779% |
| TPP1 | 1.07E+05 | 1.78E+06 | 1659% | RHPN2 | 4.02E+04 | 3.26E+05 | 810% |
| TPM4 | 1.18E+06 | 1.01E+07 | 861% | RAB3D | 1.21E+06 | 2.51E+06 | 208% |
| TPM3 | 5.41E+05 | 5.47E+06 | 1011% | RAB32 | 3.11E+05 | 9.02E+05 | 290% |
| TPM1 | 7.84E+05 | 8.89E+06 | 1133% | RAB2A | 4.34E+05 | 1.30E+06 | 299% |
| TMEM30A | 6.52E+05 | 1.30E+06 | 199% | RAB21 | 1.86E+05 | 5.33E+05 | 287% |
| TC2N | 3.25E+05 | 6.62E+05 | 204% | RAB14 | 9.07E+04 | 6.77E+05 | 747% |
| SYPL1 | 1.15E+06 | 4.56E+06 | 395% | PTPN6 | 2.14E+05 | 5.22E+05 | 244% |
| SUSD2 | 1.06E+05 | 1.10E+06 | 1035% | PSMB8 | 3.37E+04 | 1.80E+06 | 5334% |
| SURF4 | 1.30E+04 | 4.05E+05 | 3120% | PSMB1 | 4.83E+04 | 2.04E+06 | 4228% |
| STOM | 1.10E+06 | 1.03E+07 | 936% | PSMA1 | 1.24E+05 | 5.35E+06 | 4316% |
| ST6GALNAC1 | 4.59E+04 | 5.24E+05 | 1141% | PRKG2 | 6.83E+04 | 2.09E+05 | 306% |
| ST13 | 7.85E+04 | 2.24E+05 | 285% | PRKCD | 2.33E+05 | 5.39E+05 | 232% |
| SQOR | 1.02E+05 | 5.26E+06 | 5151% | PRKACA | 1.04E+05 | 3.14E+05 | 302% |
| SPTBN1 | 8.78E+05 | 1.07E+07 | 1215% | PRDX6 | 7.47E+05 | 1.58E+06 | 212% |
| SPPL2A | 5.25E+05 | 1.63E+06 | 311% | PRDX5 | 1.07E+06 | 2.42E+06 | 226% |
| SMPD3 | 2.90E+04 | 2.51E+05 | 865% | PRDX2 | 7.62E+04 | 7.32E+05 | 961% |
| SLC9A3 | 7.73E+05 | 1.36E+06 | 176% | PRDX1 | 9.01E+05 | 2.19E+06 | 243% |
| SLC6A14 | 5.74E+04 | 4.43E+05 | 771% | PPP1CB | 3.41E+05 | 5.30E+05 | 156% |
| SLC46A1 | 3.25E+05 | 1.31E+06 | 402% | PNP | 8.18E+04 | 4.48E+05 | 547% |
| SLC44A1 | 3.03E+05 | 7.61E+05 | 251% | PLEC | 1.52E+04 | 2.77E+05 | 1824% |
| SLC36A1 | 4.04E+04 | 8.38E+04 | 208% | PLA2G2A | 4.06E+07 | 1.93E+08 | 475% |
| SLC2A5 | 1.23E+06 | 3.79E+06 | 309% | PLA2G10 | 8.31E+04 | 3.12E+05 | 376% |
| SLC25A5 | 7.69E+05 | 5.57E+06 | 724% | PKM | 4.20E+05 | 6.51E+06 | 1549% |
| SLC25A4 | 3.64E+05 | 1.38E+06 | 380% | PICALM | 2.30E+05 | 4.53E+05 | 197% |
| SLC16A1 | 1.15E+04 | 5.96E+04 | 520% | PGK1 | 7.76E+05 | 2.25E+06 | 290% |
| SLC15A4 | 1.32E+05 | 8.10E+05 | 612% | PGD | 2.05E+05 | 3.79E+06 | 1852% |
| SLC15A1 | 5.43E+05 | 1.09E+06 | 201% | PFN1 | 6.09E+06 | 2.25E+07 | 369% |
| SERPINF2 | 7.45E+04 | 3.49E+05 | 468% | PCYOX1 | 5.97E+04 | 8.43E+05 | 1411% |
| SERPINA7 | 2.51E+04 | 5.53E+05 | 2207% | PCNT | 7.26E+04 | 3.77E+05 | 520% |
| SDHB | 7.66E+04 | 1.17E+06 | 1528% | PCBP1 | 3.70E+05 | 1.03E+06 | 279% |
| SDHA | 2.70E+04 | 6.67E+05 | 2472% | PAFAH1B1 | 3.17E+04 | 1.32E+05 | 416% |
| SCARB2 | 2.06E+06 | 4.72E+06 | 229% | P2RX4 | 3.95E+05 | 8.94E+05 | 226% |
| SAFB2 | 1.88E+07 | 5.66E+07 | 301% | OTUB1 | 1.73E+05 | 3.27E+05 | 189% |

**[Table 11]**

| Protein | Quantified value of healthy human fecal exosomes (average) | Quantified value of colorectal cancer patient fecal exosomes (average) | Ratio | Protein | Quantified value of healthy human fecal exosomes (average) | Quantified value of colorectal cancer patient fecal exosomes (average) | Ratio |
|---|---|---|---|---|---|---|---|
| NPEPPS | 1.22E+05 | 6.10E+05 | 502% | GSTA1 | 1.06E+05 | 2.02E+05 | 191% |
| NIBAN2 | 2.65E+05 | 4.78E+05 | 180% | GSDME | 1.64E+05 | 1.18E+06 | 722% |
| NEDD4L | 3.66E+05 | 8.59E+05 | 235% | GRN | 5.81E+05 | 1.28E+06 | 221% |
| NCSTN | 2.44E+06 | 4.27E+06 | 175% | GPI | 3.56E+05 | 1.43E+07 | 4012% |
| MYOF | 2.82E+05 | 1.09E+06 | 386% | GPD2 | 2.89E+05 | 1.08E+06 | 372% |
| MYO1E | 4.56E+05 | 8.43E+05 | 185% | GPA33 | 7.39E+05 | 1.91E+06 | 258% |
| MYO1C | 1.30E+06 | 2.51E+06 | 193% | GP2 | 2.61E+07 | 4.69E+07 | 179% |
| MYO1B | 3.09E+05 | 5.57E+05 | 181% | GNAI2 | 7.69E+04 | 1.10E+06 | 1433% |
| MYH9 | 1.36E+06 | 4.91E+06 | 360% | GMDS | 1.31E+05 | 5.24E+06 | 4003% |
| MYH14 | 9.68E+04 | 8.34E+05 | 862% | GHITM | 5.77E+05 | 2.62E+06 | 454% |
| MUC4 | 3.35E+06 | 1.03E+07 | 309% | GFM2 | 8.46E+04 | 1.75E+06 | 2069% |
| MUC3A | 5.96E+05 | 6.01E+06 | 1008% | GDPD3 | 1.11E+06 | 2.75E+06 | 249% |
| MT-CYB | 1.91E+05 | 3.45E+05 | 181% | GAPDH | 7.88E+06 | 1.72E+07 | 218% |
| MIS12 | 2.41E+05 | 7.55E+05 | 313% | FUT3 | 1.23E+05 | 4.17E+05 | 340% |
| MIF | 6.52E+04 | 5.94E+05 | 911% | FLOT2 | 2.85E+05 | 5.56E+05 | 195% |
| MGST3 | 1.78E+05 | 1.50E+06 | 839% | FLOT1 | 1.80E+05 | 3.46E+05 | 192% |
| MGLL | 3.97E+05 | 8.74E+05 | 220% | F11R | 3.04E+04 | 1.06E+06 | 3470% |
| MEP1A | 3.27E+07 | 2.11E+08 | 646% | EVC2 | 6.90E+04 | 1.81E+06 | 2616% |
| MAOB | 5.32E+04 | 6.90E+05 | 1297% | ETHE1 | 1.41E+05 | 4.46E+05 | 317% |
| MAOA | 1.15E+05 | 3.32E+06 | 2883% | ESD | 5.87E+04 | 1.45E+05 | 247% |
| LYN | 9.69E+05 | 2.21E+06 | 228% | ERLIN2 | 5.18E+04 | 3.72E+05 | 718% |
| LSM2 | 2.83E+04 | 6.88E+04 | 243% | EPCAM | 4.58E+05 | 6.78E+06 | 1482% |
| LGALS4 | 3.09E+07 | 4.76E+07 | 154% | ENPP4 | 6.48E+04 | 1.54E+06 | 2372% |
| LGALS3BP | 5.16E+06 | 4.91E+07 | 952% | ENO1 | 7.02E+05 | 2.42E+06 | 345% |
| LDHA | 9.73E+05 | 1.78E+07 | 1827% | EIF5A | 2.90E+05 | 4.62E+05 | 160% |
| LAMP1 | 2.33E+05 | 5.56E+06 | 2383% | EIF4A1 | 3.26E+05 | 1.47E+06 | 451% |
| ITGA6 | 1.90E+05 | 9.77E+05 | 515% | EEF2 | 4.69E+05 | 1.90E+06 | 406% |
| ITGA3 | 1.14E+05 | 6.54E+05 | 573% | EBP | 9.83E+04 | 6.68E+05 | 679% |
| ITGA1 | 6.28E+04 | 2.00E+05 | 318% | DSTN | 4.59E+05 | 7.64E+05 | 166% |
| IGLV7-46 | 4.85E+05 | 1.28E+06 | 264% | DNPEP | 2.80E+05 | 6.36E+05 | 227% |
| IGKV1-39 | 1.25E+05 | 3.95E+05 | 315% | DLD | 7.52E+04 | 2.85E+06 | 3788% |
| IGHM | 1.01E+08 | 4.43E+08 | 438% | DDOST | 1.07E+05 | 1.87E+05 | 175% |
| IGHG4 | 5.65E+04 | 3.54E+06 | 6268% | CYFIP1 | 3.59E+05 | 5.66E+05 | 158% |
| IGHG2 | 1.76E+07 | 1.97E+08 | 1114% | CSRP1 | 7.88E+04 | 1.88E+05 | 238% |
| IDH1 | 1.57E+05 | 7.68E+05 | 490% | CSNK1A1 | 3.13E+05 | 5.27E+05 | 168% |
| HTATIP2 | 5.25E+04 | 3.00E+05 | 572% | CPNE3 | 6.95E+05 | 1.74E+06 | 250% |
| HSPA5 | 4.91E+05 | 2.09E+06 | 426% | CORO1B | 3.72E+04 | 1.05E+05 | 284% |
| HSP90AB1 | 5.15E+05 | 2.55E+06 | 495% | CNDP2 | 9.80E+04 | 4.32E+05 | 440% |
| HSD17B4 | 2.27E+05 | 5.70E+05 | 251% | CLTC | 3.86E+04 | 1.11E+06 | 2868% |
| HNRNPA2B1 | 1.43E+05 | 9.78E+05 | 685% | CLIC1 | 1.60E+06 | 3.16E+06 | 198% |
| HM13 | 1.07E+05 | 4.86E+05 | 456% | CHP2 | 2.18E+05 | 8.28E+05 | 380% |
| HLA-DMA | 2.43E+05 | 5.04E+05 | 207% | CHMP2A | 1.48E+05 | 4.13E+05 | 279% |
| HARS1 | 1.03E+05 | 2.67E+05 | 260% | CEACAM5 | 1.38E+08 | 2.74E+08 | 198% |
| H2BC12 | 1.96E+05 | 1.30E+07 | 6629% | CEACAM1 | 2.99E+07 | 4.84E+07 | 162% |
| H1-4 | 2.51E+05 | 6.29E+05 | 250% | CD151 | 2.35E+05 | 2.23E+06 | 947% |
| GSTP1 | 5.27E+05 | 1.07E+06 | 202% | CASQ1 | 2.20E+04 | 1.94E+05 | 881% |

**[Table 12]**

| Protein | Quantified value of healthy human fecal exosomes (average) | Quantified value of colorectal cancer patient fecal exosomes (average) | Ratio |
|---|---|---|---|
| CAPZB | 1.31E+05 | 1.66E+06 | 1268% |
| CAPZA1 | 7.19E+04 | 1.97E+05 | 275% |
| CAPN1 | 1.00E+05 | 7.14E+05 | 712% |
| CAP1 | 1.32E+05 | 4.25E+06 | 3232% |
| C9 | 3.09E+05 | 1.78E+07 | 5749% |
| BLVRB | 1.06E+05 | 2.82E+05 | 267% |
| B3GNT7 | 9.28E+04 | 7.39E+05 | 797% |
| B2M | 1.89E+04 | 2.02E+06 | 10676% |
| ATP9A | 6.38E+04 | 5.12E+05 | 803% |
| ATP1B3 | 1.19E+04 | 1.77E+05 | 1486% |
| ATP1B1 | 1.18E+06 | 2.68E+06 | 228% |
| ATP1A1 | 1.59E+06 | 5.27E+06 | 331% |
| ASAH1 | 7.90E+05 | 5.93E+06 | 751% |
| ARPC5 | 5.76E+04 | 8.98E+05 | 1560% |
| ARPC2 | 9.33E+04 | 1.45E+06 | 1551% |
| ARL1 | 1.93E+05 | 8.63E+05 | 447% |
| ARHGDIA | 1.94E+05 | 1.09E+06 | 561% |
| AP2M1 | 3.30E+04 | 1.62E+05 | 491% |
| AP2A1 | 1.81E+05 | 3.54E+05 | 196% |
| AP1B1 | 4.98E+04 | 2.50E+05 | 501% |
| ANXA4 | 4.18E+06 | 9.27E+06 | 222% |
| ANXA3 | 1.67E+05 | 5.50E+06 | 3284% |
| ANO6 | 8.60E+03 | 1.45E+05 | 1682% |
| ALDOA | 7.57E+05 | 2.13E+06 | 282% |
| ALDH3B1 | 2.73E+05 | 8.68E+05 | 319% |
| AGR3 | 6.44E+04 | 2.48E+05 | 385% |
| AGR2 | 3.62E+04 | 9.57E+05 | 2640% |
| ACTR3 | 1.43E+05 | 2.13E+06 | 1487% |
| ACTR2 | 1.58E+05 | 1.17E+06 | 740% |
| ACTN4 | 1.71E+05 | 1.13E+06 | 663% |
| ACAA1 | 3.14E+04 | 5.05E+05 | 1608% |
| ABRACL | 7.23E+04 | 1.40E+05 | 194% |

**[Table 13]**

| Protein | Quantified value of colorectal cancer patient fecal exosomes (average) | Protein | Quantified value of colorectal cancer patient fecal exosomes (average) | Protein | Quantified value of colorectal cancer patient fecal exosomes (average) |
|---|---|---|---|---|---|
| YARS1 | 9.19E+03 | STXBP6 | 2.03E+05 | RRAGB | 5.25E+04 |
| XRCC6 | 1.29E+05 | STIP1 | 4.17E+06 | RPS9 | 9.02E+05 |
| XRCC5 | 1.17E+05 | STAT3 | 9.10E+05 | RPS4X | 1.42E+05 |
| XPO1 | 1.20E+05 | STAT1 | 1.43E+05 | RPS3A | 8.43E+05 |
| WDR44 | 4.29E+04 | SSR4 | 4.02E+05 | RPS24 | 6.49E+05 |
| VPS35 | 6.48E+05 | SSR1 | 9.41E+05 | RPS18 | 4.68E+05 |
| VPS29 | 1.11E+05 | SRSF3 | 1.38E+05 | RPS17 | 1.34E+05 |
| VPS26A | 3.91E+05 | SRM | 1.09E+05 | RPS14 | 1.07E+05 |
| VKORC1L1 | 2.88E+04 | SPCS3 | 1.85E+05 | RPS13 | 5.21E+05 |
| VBP1 | 2.27E+04 | SPAST | 5.47E+04 | RPN1 | 3.96E+05 |
| VAMP7 | 5.74E+04 | SORL1 | 1.94E+05 | RPLP0 | 4.76E+05 |
| USP15 | 5.72E+04 | SORD | 5.74E+04 | RPL7 | 3.60E+05 |
| USP14 | 4.97E+04 | SOD2 | 7.72E+04 | RPL6 | 4.41E+05 |
| USO1 | 4.46E+04 | SNX33 | 3.74E+04 | RPL5 | 2.55E+05 |
| UQCRC2 | 2.26E+05 | SNRPA1 | 4.10E+04 | RPL4 | 7.07E+05 |
| UQCRC1 | 4.10E+05 | SNRNP70 | 2.82E+04 | RPL3 | 1.94E+05 |
| UPF1 | 2.48E+04 | SND1 | 2.05E+05 | RPL28 | 3.31E+05 |
| UGGT1 | 7.52E+04 | SLK | 4.54E+04 | RPL27A | 3.73E+05 |
| UBA1 | 2.31E+05 | SLC6A6 | 5.17E+04 | RPL24 | 1.14E+05 |
| TUFM | 2.31E+04 | SLC3A2 | 3.02E+05 | RPL21 | 2.92E+05 |
| TPP2 | 4.77E+05 | SLC35B2 | 2.85E+04 | RPL18A | 1.77E+05 |
| TPMT | 6.85E+04 | SLC2A3 | 2.68E+06 | RPL18 | 1.14E+06 |
| TOP2B | 3.37E+04 | SLC2A1 | 4.55E+05 | RPL17 | 3.91E+04 |
| TMEM33 | 1.03E+05 | SLC25A11 | 4.36E+05 | RPL14 | 9.64E+05 |
| TMEM205 | 2.69E+05 | SLC16A3 | 5.37E+05 | RPL13A | 4.44E+05 |
| TMED9 | 5.19E+05 | SLC12A9 | 2.22E+04 | RPL13 | 1.38E+05 |
| TMED7 | 5.94E+05 | SLC11A2 | 1.67E+05 | RPL12 | 1.38E+05 |
| TMED5 | 1.15E+05 | SIRPA | 1.10E+06 | RPL10A | 3.36E+05 |
| TMED4 | 1.57E+05 | SIL1 | 1.35E+05 | RNF149 | 1.12E+05 |
| TMED2 | 4.11E+05 | SIGMAR1 | 9.63E+05 | REEP6 | 8.45E+04 |
| TMED10 | 1.45E+06 | SHMT2 | 1.98E+05 | RCC2 | 2.37E+05 |
| TM9SF4 | 3.52E+05 | SHMT1 | 1.62E+05 | RBBP4 | 8.75E+04 |
| TM9SF3 | 7.40E+05 | SF3B3 | 2.86E+06 | RARS1 | 1.50E+05 |
| TM9SF2 | 4.51E+05 | SERPINB5 | 2.41E+05 | RACK1 | 2.17E+06 |
| TM4SF20 | 7.45E+04 | SERPINA6 | 8.82E+05 | RABGGTA | 3.30E+04 |
| TKT | 3.51E+06 | SEPTIN7 | 1.22E+05 | QARS1 | 2.06E+05 |
| THEMIS2 | 5.76E+04 | SELENOP | 1.73E+05 | PYGB | 3.52E+05 |
| TEKT2 | 3.25E+05 | SEC31A | 1.12E+06 | PTPRE | 2.31E+04 |
| TBXAS1 | 6.74E+04 | SEC24D | 9.93E+04 | PTK2B | 1.08E+05 |
| TARS1 | 1.81E+05 | SEC24C | 4.23E+04 | PTGES3 | 2.73E+05 |
| TARDBP | 9.70E+04 | SEC23B | 7.54E+04 | PSMF1 | 2.34E+05 |
| SYNGR2 | 1.75E+06 | SEC23A | 3.64E+05 | PSMD7 | 6.74E+04 |
| SYK | 1.65E+05 | SACM1L | 3.68E+04 | PSMD6 | 8.54E+04 |
| SUCLG2 | 7.11E+04 | RUVBL2 | 3.91E+04 | PSMD3 | 2.50E+05 |
| SUCLG1 | 1.23E+04 | RRM2 B | 5.60E+04 | PSMD2 | 1.33E+05 |
| SUCLA2 | 1.40E+05 | RRAGC | 7.46E+04 | PSMD13 | 1.24E+05 |

**[Table 14]**

| Protein | Quantified value of colorectal cancer patient fecal exosomes (average) | Protein | Quantified value of colorectal cancer patient fecal exosomes (average) | Protein | Quantified value of colorectal cancer patient fecal exosomes (average) |
|---|---|---|---|---|---|
| PSMD12 | 2.20E+05 | NPC1 | 9.30E+04 | HTT | 1.64E+05 |
| PSMD1 | 1.32E+05 | NOSTRIN | 2.27E+05 | HSPH1 | 5.88E+04 |
| PSMC5 | 8.55E+04 | NNT | 4.89E+05 | HSPA9 | 3.43E+05 |
| PSMC3 | 6.80E+04 | NMT1 | 8.81E+04 | HSPA4 | 4.38E+05 |
| PSMC2 | 2.16E+05 | NIPSNAP2 | 3.39E+05 | HSP90B1 | 2.82E+06 |
| PRXL2A | 7.65E+04 | NIBAN1 | 9.31E+04 | HSDL2 | 2.89E+04 |
| PRPS1 | 3.81E+05 | NDUFV1 | 2.11E+05 | HSD17B11 | 2.94E+05 |
| PRPF19 | 6.83E+04 | NDUFB10 | 5.47E+04 | HSD17B10 | 4.86E+04 |
| PRMT1 | 6.25E+04 | NCKAP1L | 9.62E+04 | HSD11B2 | 2.25E+05 |
| PRKDC | 8.47E+04 | NARS1 | 1.73E+05 | HNRNPUL2 | 1.80E+05 |
| PRKCB | 1.27E+04 | MVD | 5.93E+04 | HNRNPU | 2.13E+05 |
| PPP2CB | 1.67E+05 | MT-CO2 | 3.24E+05 | HNRNPK | 2.69E+05 |
| PPIB | 1.17E+06 | MOGS | 5.45E+04 | HNRNPC | 1.07E+06 |
| POR | 3.74E+05 | MOB1B | 2.10E+05 | HMGCS2 | 1.74E+06 |
| PON1 | 5.01E+05 | MICAL1 | 1.28E+05 | HMGB1 | 1.13E+05 |
| POLR2H | 2.38E+04 | MGST2 | 1.47E+05 | HLA-B | 2.59E+04 |
| PLTP | 1.95E+06 | MGST1 | 3.91E+05 | HKDC1 | 7.27E+04 |
| PLP2 | 1.03E+05 | MFGE8 | 1.58E+06 | HK2 | 5.43E+04 |
| PLEKHA1 | 2.52E+04 | MCCC2 | 8.06E+04 | HK1 | 2.47E+05 |
| PLCG2 | 2.77E+04 | MAT2A | 2.94E+05 | HIBCH | 3.67E+05 |
| PITPNA | 1.49E+05 | MAPK14 | 2.15E+04 | HCK | 8.93E+04 |
| PIP4P2 | 7.87E+04 | MAPK1 | 4.36E+05 | HADHB | 3.33E+05 |
| PHLDA3 | 2.87E+04 | MAN1A1 | 4.09E+05 | HADHA | 1.34E+06 |
| PHB2 | 6.71E+05 | MACC1 | 4.16E+04 | HADH | 1.28E+05 |
| PGAP6 | 6.34E+04 | M6PR | 1.32E+05 | H1-5 | 1.96E+05 |
| PFKP | 1.18E+05 | LYPLAL1 | 4.56E+04 | H1-0 | 1.23E+05 |
| PDLIM1 | 1.12E+04 | LTA4H | 1.62E+06 | GYS1 | 7.39E+04 |
| PCK2 | 1.60E+06 | LNPEP | 4.10E+05 | GSPT1 | 8.39E+04 |
| PCCA | 1.95E+04 | LBR | 4.08E+05 | GSK3A | 7.10E+04 |
| PCBP2 | 2.42E+05 | LARS1 | 1.81E+04 | GRTP1 | 1.46E+05 |
| PC | 2.28E+05 | KDELR2 | 4.49E+05 | GRK2 | 4.90E+04 |
| PARP4 | 1.72E+06 | KDELR1 | 3.47E+05 | GPLD1 | 1.32E+06 |
| PAK4 | 1.51E+04 | KARS1 | 1.11E+05 | GPD1 | 6.79E+04 |
| PAICS | 3.01E+05 | JAKMIP3 | 3.47E+06 | GOT2 | 1.41E+06 |
| PABPC1 | 2.92E+04 | ITGAV | 1.70E+05 | GOLGB1 | 1.86E+05 |
| PAAF1 | 2.72E+06 | ITGA2 | 4.59E+05 | GNPNAT1 | 3.50E+04 |
| PA2G4 | 1.90E+05 | IQGAP2 | 3.75E+04 | GMPPB | 2.29E+05 |
| OGDH | 5.40E+04 | IMPDH2 | 3.97E+05 | GDI1 | 2.29E+05 |
| OCIAD2 | 1.06E+05 | ILF2 | 1.52E+05 | GCLM | 1.28E+05 |
| OCIAD1 | 2.06E+05 | IL18R1 | 6.80E+04 | GCLC | 2.45E+04 |
| OAT | 2.64E+05 | IGKV1-12 | 1.67E+05 | GBE1 | 2.44E+05 |
| OAF | 3.61E+05 | IDH3B | 1.66E+05 | GATD3B | 1.98E+04 |
| NXF1 | 1.18E+04 | IDH3A | 1.10E+05 | GART | 3.62E+04 |
| NT5C2 | 9.50E+04 | IDH2 | 5.21E+05 | GARS1 | 1.63E+05 |
| NSDHL | 9.87E+04 | IDE | 6.40E+04 | GANAB | 4.02E+05 |
| NQO1 | 1.75E+05 | IARS1 | 6.19E+04 | GALNT2 | 3.53E+05 |

**[Table 15]**

| Protein | Quantified value of colorectal cancer patient fecal exosomes (average) | Protein | Quantified value of colorectal cancer patient fecal exosomes (average) | Protein | Quantified value of colorectal cancer patient fecal exosomes (average) |
|---|---|---|---|---|---|
| GALNT1 | 9.73E+04 | CPT2 | 1.26E+05 | ATP5MF | 5.90E+05 |
| G6PD | 1.05E+06 | CPT1A | 1.75E+05 | ATP5F1A | 4.34E+06 |
| FGR | 4.76E+05 | CPB2 | 7.52E+04 | ATP2A2 | 2.79E+05 |
| FETUB | 1.74E+05 | COX5B | 2.84E+05 | ATP11B | 4.27E+04 |
| FCN3 | 2.95E+06 | CORO7 | 7.60E+04 | ATIC | 4.74E+05 |
| FBXO7 | 7.96E+04 | CORO1C | 9.18E+04 | ATG3 | 6.27E+04 |
| FBXO6 | 4.62E+04 | COPG1 | 1.81E+05 | ASL | 2.12E+05 |
| FASN | 2.50E+05 | COPB2 | 7.21E+05 | ARPC3 | 5.97E+05 |
| FARSB | 2.36E+04 | COPA | 2.95E+05 | ARPC1B | 9.67E+05 |
| F3 | 3.46E+04 | CMPK1 | 3.01E+04 | ARHGAP1 | 9.76E+04 |
| ETFDH | 2.19E+05 | CLPX | 1.04E+05 | ARCN1 | 2.82E+05 |
| ETF1 | 8.15E+04 | CLINT1 | 9.64E+03 | APRT | 4.83E+05 |
| ERO1A | 3.35E+05 | CLDN7 | 1.64E+05 | APPL2 | 4.44E+04 |
| ERAP2 | 4.12E+05 | CHST5 | 1.60E+05 | APPL1 | 6.48E+04 |
| ERAP1 | 4.04E+05 | CEMIP2 | 6.83E+04 | APMAP | 4.66E+06 |
| ENTPD1 | 2.67E+05 | CDCP1 | 8.06E+04 | APIP | 1.68E+05 |
| EML4 | 8.71E+04 | CCT8 | 8.86E+04 | APEX1 | 2.04E+05 |
| EIF3L | 5.06E+04 | CCT7 | 9.13E+04 | AP1S1 | 1.67E+05 |
| EIF3I | 6.93E+04 | CCT6A | 2.76E+05 | AP1M1 | 1.33E+05 |
| EIF3D | 5.55E+04 | CCT5 | 1.13E+05 | AP1G1 | 1.32E+05 |
| EIF3CL | 4.00E+04 | CCT4 | 2.86E+05 | ALOX15 | 6.19E+05 |
| EIF3B | 1.93E+05 | CCT3 | 4.59E+05 | ALDH9A1 | 5.64E+05 |
| EIF2S3 | 2.27E+05 | CCT2 | 4.39E+04 | ALDH16A1 | 7.93E+04 |
| EIF2S1 | 1.38E+05 | CASP6 | 4.67E+04 | AKR1C3 | 2.50E+05 |
| EFR3A | 4.13E+04 | C8G | 1.30E+06 | AIMP1 | 2.04E+04 |
| EEF1G | 2.62E+06 | C8B | 1.76E+06 | AIFM1 | 4.60E+05 |
| DYNC1H1 | 2.60E+05 | C7 | 3.75E+06 | AHSA1 | 8.88E+04 |
| DPYSL2 | 9.19E+04 | BUB3 | 1.74E+05 | AGPS | 4.91E+04 |
| DOP1B | 1.46E+05 | BST2 | 1.82E+05 | AGO2 | 3.16E+04 |
| DNM2 | 9.38E+05 | BSG | 7.05E+05 | ADSS2 | 2.51E+05 |
| DNAJC13 | 3.10E+04 | BPHL | 1.68E+04 | ADGRG3 | 2.07E+05 |
| DNAJB11 | 9.31E+04 | BLVRA | 1.23E+05 | ADGRE5 | 1.36E+05 |
| DLAT | 3.13E+05 | BAX | 3.68E+04 | ADAM10 | 4.93E+05 |
| DHX9 | 3.35E+05 | BACE1 | 4.14E+04 | ACSL5 | 2.77E+05 |
| DHRS9 | 1.24E+06 | B3GNT8 | 2.22E+04 | ACSL1 | 5.60E+05 |
| DHRS7 | 9.94E+05 | B3GNT3 | 6.24E+04 | ACOT7 | 1.70E+04 |
| DDX1 | 2.69E+04 | ATP8A1 | 8.01E+04 | ACOT11 | 4.56E+04 |
| DDC | 8.18E+05 | ATP6V1H | 2.90E+04 | ACLY | 3.31E+05 |
| DARS1 | 1.03E+05 | ATP6V1G1 | 2.70E+04 | ACADS | 1.16E+05 |
| DAD1 | 1.22E+05 | ATP6V1E1 | 3.57E+04 | ACADM | 2.32E+05 |
| CYFIP2 | 6.86E+04 | ATP6V1A | 7.54E+04 | ACAA2 | 6.61E+05 |
| CYB5R3 | 1.55E+05 | ATP6V0D1 | 1.11E+05 | ABRAXAS2 | 3.87E+04 |
| CYB5B | 3.02E+05 | ATP6V0A1 | 7.02E+04 | ABCG8 | 3.95E+04 |
| CSK | 3.94E+05 | ATP5PO | 5.33E+04 | ABCF1 | 1.17E+04 |
| CRYZ | 1.85E+05 | ATP5PB | 8.24E+04 | | |
| CRYL1 | 1.56E+05 | ATP5MG | 7.79E+05 | | |

### <Test Example 6> Comparison of colorectal cancer-related proteins in fecal exosomes and in total feces 1

From the proteins described in Fig. 11 obtained in Test Example 5 above, proteins for which the quantified value (content ratio) in colorectal cancer patient fecal exosomes (fEV) was two times or more compared to the quantified value in colorectal cancer patient total feces (feces) were selected; among them, for 5 types as examples (LAMP1, ATP5F1A, LGALS3BP, DLD, ENPP4), the quantified value (average value) in healthy human fecal exosomes and the quantified value (average value) in colorectal cancer patient fecal exosomes, and the quantified value (average value) in healthy human total feces and the quantified value (average value) in colorectal cancer patient total feces were compared, respectively. Fig. 12 shows (a) a graph comparing the quantified value in healthy human fecal exosomes (HC) and the quantified value in colorectal cancer patient fecal exosomes (CC), respectively, and (b) a graph comparing the quantified value in healthy human total feces (HC) and the quantified value in colorectal cancer patient total feces (CC), respectively.

From the proteins described in Fig. 11 obtained in Test Example 5 above, proteins that were not detected in colorectal cancer patient total feces (feces) but were detected only in colorectal cancer patient fecal exosomes (fEV) were selected; among them, for 5 types as examples (MAPK1, PCYOX1, SMPD3, VILL, RPS7), the quantified value (average value) in healthy human fecal exosomes and the quantified value (average value) in colorectal cancer patient fecal exosomes, and the quantified value (average value) in healthy human total feces and the quantified value (average value) in colorectal cancer patient total feces were compared, respectively. Fig. 13 shows (a) a graph comparing the quantified value in healthy human fecal exosomes (HC) and the quantified value in colorectal cancer patient fecal exosomes (CC), respectively, and (b) a graph comparing the quantified value in healthy human total feces (HC) and the quantified value in colorectal cancer patient total feces (CC), respectively. In Figs. 12 and 13, "*" indicates p<0.05 in the Mann-Whitney U test, and "ns" indicates no significant difference.

As shown in Figs. 12 and 13, for the colorectal cancer-related proteins concentrated in colorectal cancer patient fecal exosomes, that is, proteins for which the quantified value (content ratio) in colorectal cancer patient fecal exosomes was two times or more the quantified value (content ratio) in colorectal cancer patient total feces, or proteins that were not detected in colorectal cancer patient total feces but were detected only in colorectal cancer patient fecal exosomes, the quantified value in each fecal exosome was significantly different between colorectal cancer patients and healthy individuals; this confirmed that it is indeed possible to detect colorectal cancer patients by detecting these colorectal cancer-related proteins from fecal exosomes, for example, using their quantified values as an indicator (Figs. 12(a) and 13(a)). On the other hand, when these proteins were compared by the quantified value in each total feces, there was no significant difference between colorectal cancer patients and healthy individuals, confirming that it is difficult to distinguish between them from total feces (Figs. 12(b) and 13(b)).

### <Test Example 7> PLS regression (Partial Least Squares regression) analysis

Pathology data from "The Human Protein Atlas database (https://www.proteinatlas.org/)" was downloaded (June 16, 2023), and colorectal cancer data was obtained from them. From the obtained data, a ratio (n/N) was calculated by dividing the total number of proteins (n) with staining levels of High, Medium, or Low by the total number of stainings (N), and those for which this ratio exceeded 0.5 were selected as colorectal cancer-related proteins. The number of colorectal cancer-related proteins selected from the database was 9723 proteins.

Next, from the proteins identified in each healthy human fecal exosome and colorectal cancer patient fecal exosome in (2) of Test Example 4, proteins that matched the 9723 proteins above were extracted; furthermore, from among these, for proteins for which the quantified value (content ratio) in healthy human fecal exosomes was two times or more compared to the quantified value in healthy human total feces, PLS regression analysis was performed for each group: the group with a molecular weight of 25 kDa or less (21 proteins), the group with a molecular weight of 25 to 75 kDa (49 proteins), and the group with a molecular weight of 75 kDa or more (14 proteins), using the quantified values in healthy human fecal exosomes (4 specimens) and the quantified values in colorectal cancer patient fecal exosomes (4 specimens), using JMP software (JMP 17.0.0, JMP Statistical Discovery LLC) and selecting the NIPALS method. A plot diagram showing the results is shown in Fig. 14(a). A similar PLS regression analysis was performed for colorectal cancer-related proteins that were not detected in healthy human total feces but were detected only in healthy human fecal exosomes. A plot diagram showing each result is shown in Fig. 14(b).

Furthermore, from among the proteins that matched the 9723 proteins above, for proteins for which the quantified value in healthy human fecal exosomes was two times or more compared to the quantified value in healthy human total feces, PLS regression analysis was performed for each group: the group of secreted proteins (Secreted protein, 18 proteins), the group of membrane proteins (Membrane protein, 46 proteins), the group of cytoplasmic proteins (Cytoplasmic protein, 56 proteins), and the group of nuclear proteins (Nuclear protein, 23 proteins), using the quantified values in healthy human fecal exosomes (4 specimens) and the quantified values in colorectal cancer patient fecal exosomes (4 specimens), using JMP software and selecting the NIPALS method. A plot diagram showing the results is shown in Fig. 15(a). A similar PLS regression analysis was performed for colorectal cancer-related proteins that were not detected in healthy human total feces but were detected only in healthy human fecal exosomes. A plot diagram showing each result is shown in Fig. 15(b).

Furthermore, from among the proteins that matched the 9723 proteins above, for colorectal cancer-related proteins for which the quantified value in colorectal cancer patient fecal exosomes was two times or more compared to the quantified value in colorectal cancer patient total feces, PLS regression analysis was performed for each group: the group with a molecular weight of 25 kDa or less (60 proteins), the group with a molecular weight of 25 to 75 kDa (186 proteins), and the group with a molecular weight of 75 kDa or more (64 proteins), using the quantified values in healthy human fecal exosomes (4 specimens) and the quantified values in colorectal cancer patient fecal exosomes (4 specimens), using JMP software and selecting the NIPALS method. A plot diagram showing the results is shown in Fig. 16(a). A similar PLS regression analysis was performed for colorectal cancer-related proteins that were not detected in colorectal cancer patient total feces but were detected only in colorectal cancer patient fecal exosomes. A plot diagram showing each result is shown in Fig. 16(b).

Furthermore, from among the proteins that matched the 9723 proteins above, for colorectal cancer-related proteins for which the quantified value in colorectal cancer patient fecal exosomes was two times or more compared to the quantified value in colorectal cancer patient total feces, PLS regression analysis was performed for each group: the group of secreted proteins (Secreted protein, 54 proteins), the group of membrane proteins (Membrane protein, 135 proteins), the group of cytoplasmic proteins (Cytoplasmic protein, 169 proteins), and the group of nuclear proteins (Nuclear protein, 77 proteins), using the quantified values in healthy human fecal exosomes (4 specimens) and the quantified values in colorectal cancer patient fecal exosomes (4 specimens), using JMP software and selecting the NIPALS method. A plot diagram showing the results is shown in Fig. 17(a). A similar PLS regression analysis was performed for colorectal cancer-related proteins that were not detected in colorectal cancer patient total feces but were detected only in colorectal cancer patient fecal exosomes. A plot diagram showing each result is shown in Fig. 17(b).

As shown in Figs. 14 to 17, for the colorectal cancer-related proteins concentrated in each fecal exosome, that is, proteins for which the quantified value (content ratio) in each fecal exosome was two times or more the quantified value (content ratio) in each total feces, or proteins that were not detected in each total feces but were detected only in each fecal exosome, the distribution trend of the quantified values in each fecal exosome separated into two groups between colorectal cancer patients and healthy individuals, regardless of the difference in protein molecular weight (Figs. 14 and 16) or the difference in protein localization (Figs. 15 and 17), and it was confirmed that it is possible to detect colorectal cancer patients using the quantified values of these colorectal cancer-related proteins as an indicator.

### <Test Example 8> Comparison between healthy human fecal exosomes and colorectal cancer patient fecal exosomes 3

First, in addition to the 9723 proteins selected in Test Example 5, 155 types of proteins that have been conventionally reported as biomarkers for colorectal cancer or as having potential to be so were further selected.

Next, from the proteins identified in each healthy human fecal exosome and colorectal cancer patient fecal exosome in (2) of Test Example 4, 57 types of proteins that matched the 155 types of proteins above were extracted; furthermore, from among these, proteome analysis by mass spectrometry was performed in the same manner as in (2) of Test Example 3 to calculate quantified values (content ratios) for proteins meeting at least one of the following: proteins that were common to healthy human fecal exosomes and healthy human total feces and for which the content ratio in healthy human fecal exosomes was two times or more (200% or more) the content ratio (quantified value) in healthy human total feces; proteins that were not detected in healthy human total feces but were detected only in healthy human fecal exosomes; proteins that were common to colorectal cancer patient fecal exosomes and colorectal cancer patient total feces and for which the content ratio in colorectal cancer patient fecal exosomes was two times or more (200% or more) the content ratio in colorectal cancer patient total feces; and proteins that were not detected in colorectal cancer patient total feces but were detected only in colorectal cancer patient fecal exosomes.

For the colorectal cancer-related proteins on which analysis was performed, the ratio (Ratio: quantified value in colorectal cancer patient fecal exosomes / quantified value in healthy human fecal exosomes × 100 (%)) between the average value of quantified values in healthy human fecal exosomes (average value of Healthy individuals 1 to 4; the quantified value of a specimen in which the protein was not identified was calculated as 0) and the average value of quantified values in colorectal cancer patient fecal exosomes (average value of Colorectal cancer 1 to 3; the quantified value of a specimen in which the protein was not identified was calculated as 0) was calculated. A list of proteins for which the ratio (Ratio) was 150% (1.5 times) or more, along with their average quantified values in each fecal exosome and the ratio (Ratio), is shown in Table 16 below. A list of colorectal cancer-related proteins on which analysis was performed that were not detected in any of specimens 1 to 4 of healthy human fecal exosomes and were detected only in at least one of the colorectal cancer patient fecal exosomes of Colorectal cancer 1 to 3, and their average quantified values in each fecal exosome (average value of Colorectal cancer 1 to 3; the quantified value of a specimen in which the protein was not identified was calculated as 0), is also shown in Table 16. In Table 16, LCP1, ANXA1, and OLFM4 were commonly detected from fecal exosomes in all colorectal cancer patient specimens (4 specimens), and FBLN1, COL6A1, TGFBI, and THBS1 were commonly detected from fecal exosomes in 2 or 3 colorectal cancer patient specimens.

**[Table 16]**

| Protein | Quantified value of healthy human fecal exosomes (average) | Quantified value of colorectal cancer patient fecal exosomes (average) | Ratio |
|---|---|---|---|
| LCP1 | 9.27E+05 | 2.12E+06 | 228% |
| ANXA1 | 1.09E+05 | 2.27E+06 | 2082% |
| OLFM4 | 3.55E+06 | 2.17E+08 | 6116% |
| FBLN1 | 0 | 2.94E+06 | - |
| COL6A1 | 0 | 2.92E+06 | - |
| TGFBI | 0 | 1.01E+06 | - |
| THBS1 | 0 | 2.86E+05 | - |

In both healthy individuals and colorectal cancer patients, colorectal cancer-related proteins are concentrated in fecal exosomes (that is, the quantified value (content ratio) in each fecal exosome is two times or more the quantified value (content ratio) in each total feces, or they are not detected in each total feces but detected only in each fecal exosome); however, as shown in Table 16, it was confirmed that in the fecal exosomes of colorectal cancer patients, many colorectal cancer-related proteins are concentrated, and the content is significantly higher than in the fecal exosomes of healthy individuals (that is, the Ratio is 150% or more). Furthermore, as shown in Table 16, there were colorectal cancer-related proteins that were not detected in healthy human fecal exosomes but were concentrated and detected only in colorectal cancer patient fecal exosomes. From these results, it was confirmed that gastrointestinal disease-related proteins (for example, colorectal cancer-related proteins) are concentrated in fecal exosomes (especially in the fecal exosomes of gastrointestinal disease patients (for example, colorectal cancer patients)).

### <Test Example 9> Comparison of colorectal cancer-related proteins in fecal exosomes and in total feces 2

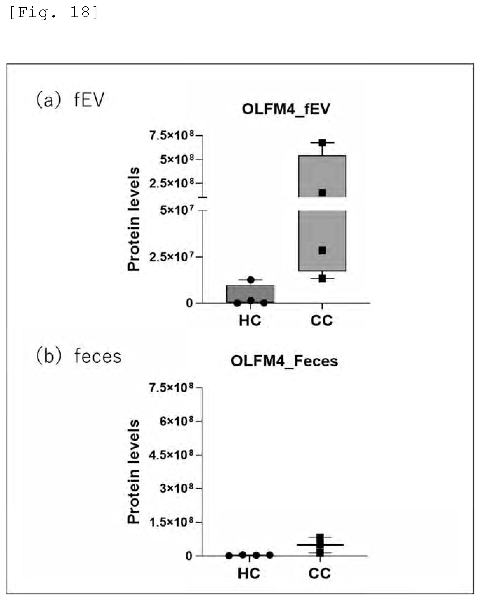

Among the 7 types of proteins described in Table 16 obtained in Test Example 8 above, for OLFM4 as an example, the quantified value (average value) in healthy human fecal exosomes and the quantified value (average value) in colorectal cancer patient fecal exosomes, and the quantified value (average value) in healthy human total feces and the quantified value (average value) in colorectal cancer patient total feces were compared, respectively. Fig. 18 shows (a: fecal exosomes (fEV)) a graph comparing the quantified value in healthy human fecal exosomes (HC) and the quantified value in colorectal cancer patient fecal exosomes (CC), respectively, and (b: total feces (feces)) a graph comparing the quantified value in healthy human total feces (HC) and the quantified value in colorectal cancer patient total feces (CC), respectively.

As shown in Fig. 18, for the colorectal cancer-related protein concentrated in colorectal cancer patient fecal exosomes, that is, OLFM4, for which the quantified value (content ratio) in colorectal cancer patient fecal exosomes was two times or more the quantified value (content ratio) in colorectal cancer patient total feces, the quantified value in fecal exosomes was significantly different between colorectal cancer patients and healthy individuals; this confirmed that it is indeed possible to detect colorectal cancer patients by detecting such a colorectal cancer-related protein from fecal exosomes, for example, using its quantified value as an indicator (Fig. 18(a)). On the other hand, when this protein was compared by the quantified value in total feces, there was no significant difference between colorectal cancer patients and healthy individuals, confirming that it is difficult to distinguish between them from total feces (Fig. 18(b)).

### <Test Example 10> Detection of colorectal cancer-related proteins by Western blotting

Among the proteins described in Fig. 11 obtained in Test Example 5 above and the proteins described in Table 16 obtained in Test Example 9 above, for 3 types as examples (OLFM4, LAMP1, LGALS3BP), detection from fecal exosomes by Western blotting was performed.

Specifically, first, the protein concentration in the healthy human fecal exosome sample (HC) and the colorectal cancer patient fecal exosome sample (CC) obtained in (1) of Test Example 4 was measured by the BCA method. From the obtained protein concentration, each fecal exosome sample was aliquoted to contain 2.5 µg of protein, mixed with purified water, SDS, and DTT, then heated at 95°C for 10 minutes to prepare a sample for Western blotting. 20 µL of the prepared sample was subjected to electrophoresis on a 10 to 20% concentration acrylamide gel, then transferred to a PVDF membrane, and reacted with anti-OLFM4 antibody (rabbit-derived, manufactured by Cell Signaling Technology), anti-LAMP1 antibody (rabbit-derived, manufactured by Cell Signaling Technology), or anti-LGALS3BP antibody (rabbit-derived, manufactured by Proteintech) overnight in the cold. Next, the membrane was reacted with HRP-labeled anti-rabbit antibody for 1 hour, then developed with ECL prime (manufactured by Cytiva), and specific bands corresponding to each antibody were detected using the iBright Imaging Systems (manufactured by Thermo Fisher Scientific).

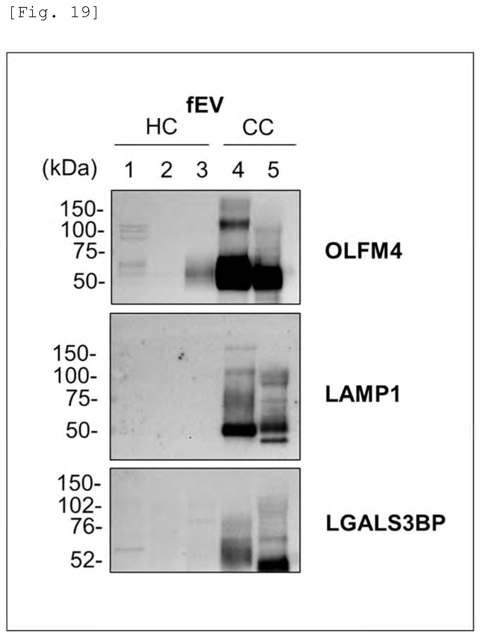

The results of Western blotting are shown in Fig. 19. As shown in Fig. 19, for all colorectal cancer-related proteins, a stronger band was detected in the colorectal cancer patient fecal exosomes (CC) than in the healthy human fecal exosomes (HC). This confirmed that by separating fecal exosomes and then detecting colorectal cancer-related proteins, it is possible to detect colorectal cancer patients even by Western blotting.

### [Industrial Applicability]

According to the present invention, a gastrointestinal disease-related protein can be efficiently detected using feces as a specimen. Furthermore, according to the present invention, it is possible to provide a method for assisting diagnosis of a gastrointestinal disease and a method for screening a diagnostic marker for a gastrointestinal disease that can efficiently detect a gastrointestinal disease-related protein using feces as a specimen, as well as a detection reagent and kit for a gastrointestinal disease-related protein for use in these methods.

## Claims

1. A method for assisting diagnosis of a gastrointestinal disease, comprising:
a step of separating fecal exosomes from feces of a subject, and
a step of detecting a gastrointestinal disease-related protein from the separated fecal exosomes,
wherein the gastrointestinal disease-related protein is a gastrointestinal disease-related protein that satisfies any one of the following conditions (a) and (b):
(a) a content ratio relative to a total amount of the fecal exosomes is 1.5 times or more a content ratio relative to a total amount of the feces,
(b) the protein is detected only from the separated fecal exosomes and is substantially not detected from the feces.

2. The method according to Claim 1, wherein the gastrointestinal disease-related protein is at least one selected from the group consisting of OLFM4, LGALS3BP, LAMP1, ATP5F1A, DLD, ENPP4, MAPK1, PCYOX1, SMPD3, VILL, and RPS7.

3. The method according to Claim 1, wherein the gastrointestinal disease is an intestinal disease.

4. The method according to Claim 1, wherein the gastrointestinal disease is colorectal cancer.

5. The method according to Claim 1, further comprising a step of quantifying the detected gastrointestinal disease-related protein.

6. A method for assisting diagnosis of a gastrointestinal disease, comprising:
a step of separating fecal exosomes from feces of a subject, and
a step of detecting a gastrointestinal disease-related protein from the separated fecal exosomes,
wherein the gastrointestinal disease-related protein is at least one selected from the group consisting of ZMPSTE24, YWHAG, UGDH, TPM4, TC2N, SUSD2, STOM, ST6GALNAC1, SPTBN1, SLC6A14, SLC46A1, SLC15A4, SERPINF2, SDHB, SDHA, SAFB2, RPS16, RNF128, RNASE3, RAB21, RAB14, PRKG2, PLEC, PLA2G10, NPEPPS, MYH14, MT-CYB, MAOA, ITGA3, IGLV7-46, HSD17B4, HARS1, H1-4, GSTA1, GMDS, GFM2, FUT3, FLOT2, DNPEP, CNDP2, CLTC, B3GNT7, B2M, ATP9A, ARPC2, AP2A1, AP1B1, AGR2, ACTR2, ABRACL, YWHAH, WDR1, VILL, VAMP8, TUBB4B, TSPAN6, TPP1, TPM3, TPM1, TMEM30A, SYPL1, SQOR, SPPL2A, SMPD3, SLC9A3, SLC44A1, SLC2A5, SLC25A5, SLC15A1, SERPINA7, SCARB2, S100A11, RPS7, RPS3, RPS23, RPS10, RPL30, RNPEP, RHPN2, RAB3D, RAB32, RAB2A, PTPN6, PSMB8, PSMB1, PSMA1, PRKCD, PRKACA, PRDX6, PRDX5, PRDX1, PPP1CB, PLA2G2A, PKM, PICALM, PGK1, PGD, PFN1, PCYOX1, PCBP1, P2RX4, NIBAN2, NEDD4L, NCSTN, MYOF, MYO1E, MYO1C, MYO1B, MYH9, MUC4, MUC3A, MGST3, MGLL, MEP1A, LYN, LGALS4, LGALS3BP, LDHA, LAMP1, IGKV1-39, IGHM, IGHG2, IDH1, HSPA5, HSP90AB1, HNRNPA2B1, HLA-DMA, H2BC12, GSTP1, GRN, GPI, GPD2, GPA33, GP2, GNAI2, GHITM, GDPD3, GAPDH, FLOT1, F11R, EPCAM, ENPP4, ENO1, EIF5A, EIF4A1, EEF2, EBP, DSTN, DLD, CYFIP1, CSNK1A1, CPNE3, CLIC1, CHMP2A, CEACAM5, CEACAM1, CD151, CAPZB, CAPN1, CAP1, C9, ATP1B1, ATP1A1, ASAH1, ARL1, ARHGDIA, ANXA4, ANXA3, ALDOA, ALDH3B1, ACTN4, ACAA1, XPO1, VPS26A, UQCRC2, UQCRC1, UBA1, TPP2, TMEM33, TMEM205, TMED4, TMED2, TMED10, TM9SF3, TM9SF2, THEMIS2, TARDBP, SYNGR2, SYK, STIP1, STAT3, STAT1, SSR4, SRSF3, SPCS3, SPAST, SOD2, SLC3A2, SLC2A3, SLC2A1, SLC16A3, SIRPA, SIL1, SERPINB5, SELENOP, SEC31A, SEC23A, RPS4X, RPS3A, RPS18, RPN1, RPL7, RPL6, RPL5, RPL4, RPL21, RPL13A, RARS1, QARS1, PTK2B, PTGES3, PSMD3, PSMD12, PSMD1, PSMC2, PRXL2A, PRPS1, PRKDC, PFKP, PCK2, PC, PAICS, OCIAD2, OAF, NT5C2, NIPSNAP2, NDUFB10, MT-CO2, MOB1B, MICAL1, MGST1, MCCC2, M6PR, LNPEP, LBR, KDELR2, KDELR1, KARS1, ITGAV, IMPDH2, IGKV1-12, IDH3B, IDH2, HTT, HSPA4, HSD17B10, HNRNPUL2, HNRNPU, HNRNPK, HMGCS2, HLA-B, HK1, HIBCH, HADHA, H1-0, GRTP1, GPD1, GMPPB, GCLM, GBE1, GARS1, GALNT2, FGR, FCN3, FASN, ERO1A, ENTPD1, EML4, EIF3D, DYNC1H1, DNM2, DLAT, DHX9, DHRS7, DARS1, CYB5B, CSK, CRYL1, CPT1A, COX5B, CORO1C, COPG1, COPA, CLDN7, CHST5, CCT4, CCT3, C8G, BSG, B3GNT3, ATP6V0D1, ATP5MG, ARPC3, ARPC1B, ARHGAP1, APMAP, AKR1C3, ADSS2, ADGRG3, ADGRE5, ACSL1, ACADM, VPS35, TMED9, TMED7, TM9SF4, STXBP6, SLC25A11, SIGMAR1, SF3B3, RPS9, RPS24, RPS13, RPLP0, RPL3, RPL18, RACK1, PYGB, PPP2CB, PPIB, PLTP, PHB2, NOSTRIN, NARS1, MAN1A1, LTA4H, JAKMIP3, ITGA2, HSD17B11, HNRNPC, GOT2, GANAB, G6PD, ERAP1, EEF1G, DOP1B, DHRS9, COPB2, ATP5MF, ATP2A2, ATIC, ARCN1, APRT, APIP, AP1S1, ALDH9A1, ADAM10, ACSL5, ACLY, TKT, SERPINA6, RPL14, PARP4, MFGE8, MAPK1, HSP90B1, GPLD1, DDC, C8B, C7, ATP5F1A, ACAA2, LCP1, ANXA1, OLFM4, FBLN1, COL6A1, TGFBI, and THBS1.

7. The method according to Claim 6, wherein the gastrointestinal disease-related protein is at least one selected from the group consisting of OLFM4, LGALS3BP, LAMP1, ATP5F1A, DLD, ENPP4, MAPK1, PCYOX1, SMPD3, VILL, and RPS7.

8. The method according to Claim 6, wherein the gastrointestinal disease is an intestinal disease.

9. The method according to Claim 6, wherein the gastrointestinal disease is colorectal cancer.

10. The method according to Claim 6, further comprising a step of quantifying the detected gastrointestinal disease-related protein.

11. A detection reagent for the gastrointestinal disease-related protein for use in the method according to any one of Claims 1 to 10.

12. A kit for use in the method according to any one of Claims 1 to 10, comprising a detection reagent for the gastrointestinal disease-related protein.

13. A method for screening a diagnostic marker for a gastrointestinal disease, comprising:
a step of separating fecal exosomes from feces,
a step of detecting a protein from the separated fecal exosomes, and
a step of selecting a candidate for a diagnostic marker for a gastrointestinal disease from the detected protein, using as an indicator that the protein satisfies any one of the following conditions (a) and (b):
(a) a content ratio relative to a total amount of the fecal exosomes is 1.5 times or more a content ratio relative to a total amount of the feces,
(b) the protein is detected only from the separated fecal exosomes and is substantially not detected from the feces.

14. The screening method according to Claim 13,
wherein the step of separating fecal exosomes from the feces and the step of detecting a protein from the separated fecal exosomes are each performed in a colorectal cancer patient and a healthy subject, and
the step of selecting a candidate for a diagnostic marker for the gastrointestinal disease is a step of selecting a candidate for a diagnostic marker for a gastrointestinal disease from the detected protein, using as an indicator that the protein satisfies any one of the following conditions (c) and (d):
(c) satisfies any one of the conditions (a) and (b) in a colorectal cancer patient,
(d) satisfies any one of the conditions (a) and (b) in a healthy subject.
